# EUROPEAN PATENT APPLICATION

(11) **EP 4 631 572 A1**
(43) Date of publication of application: **15.10.2025**
(21) Application number: 24169759.8
(22) Date of filing: 11.04.2024
(51) Int. Cl.: A61P 35/00, C07K 16/28

(54) **TMEM219 ANTIBODIES FOR MEDICAL USE**

(71) Applicant: Enthera S.r.l., 20122 Milano (MI) (IT)
(72) Inventor: AMABILE, Giovanni, 20122 Milano (MI) (IT); DANESE, Silvio, 20122 Milano (MI) (IT); MARIN, Virna, 20122 Milano (MI) (IT); ZANGARINI, Monique, 20122 Milano (MI) (IT)
(74) Representative: Pace Napoleone, Maria

(57) **Abstract**

The present invention relates to antibodies or antigen binding fragments thereof that bind to TMEM219 receptor for use in the treatment and/or prevention of colorectal cancer. Pharmaceutical compositions comprising said antibody for use in the treatment and/or prevention of colorectal cancer are also an object of the invention.

## Description

### TECHNICAL FIELD

The present invention relates to antibodies or antigen binding fragments thereof that bind specifically to the IGFBP3 receptor, namely TMEM219, for use in the treatment of colorectal cancer.

### BACKGROUND ART

Colorectal cancer (CRC) represents the third most commonly diagnosed type of cancer both in males and females (6% of all cancer subtypes) after lung (11.4%), breast cancer in women (11.6%) and prostate cancer in men (7.3%). However, colorectal cancer is also the second in terms of mortality (9.4%) after lung (18%) and the incidence is increasing globally despite a substantial geographical variety between different countries (1). In fact, colorectal cancer can be considered a marker of a country's socioeconomical development given that the most implicated risk factors, apart from family history, are linked to lifestyle and unhealthy dietary patterns such as high alcohol, red meat and processed food consumption, cigarette smoking, low physical activity, hyperlipidemia, obesity and diabetes (1,2,3).

Recent studies highlighted how CRC incidence in some countries has decreased from 1980s to 2019 linking it to a change in lifestyle, recommended colonoscopy screening and subsequent early lesion removal (4). On the other hand, even though the trend has improved for adults above 50 years, CRC new diagnoses are increasing for adults aged <50 years (1,4) as well as the incidence over the years has been constantly rising in many countries in Eastern Europe, Southeastern and South-Central Asia, and South America (1), probably due to a shift towards a western lifestyle (2).

Chronic inflammation as the one that occurs in inflammatory bowel disease (IBD) is generally recognised as an important risk factor for the development of colitis-associated colorectal cancer (CAC), a specific type of CRC (5). In fact, data show that IBD patients present both a higher risk of CAC/CRC development (2-fold increase) and an overall worse outcome compared to non-IBD patients (1.7-fold increase of death cases) (5). Over the years, mouse models, such as the azoxymethane/dextran sulfate sodium model (AOM/DSS model), have been considered very informative to investigate the tumorigenesis. In order for it to happen, two events are necessary: first a tumor-initiating event that involves mutations or epigenetic alterations and lead to oncogene hyperactivation or inactivation of tumor suppressor genes. This step is usually followed by tumor promotion, in which the cells harbouring those mutations outgrow the healthy ones (6).

Inflammation can trigger a series of detrimental mechanisms, such as DNA damage initiation and epigenetic silencing of important tumor suppressor genes in intestinal epithelial cells (IECs) (6). Moreover, chronic inflammation increases oxidative stress, leads to exposure to genotoxic compounds released by inflammatory cells, causes the intestinal barrier breakdown which leads to the interaction between IECs and pathogenic microorganisms (6). Finally, chronic inflammation triggers excessive tissue regeneration and dedifferentiation of non-stem cells into stem-like ones as a defence mechanism due to the presence of damaged tissue. This leads to tumor promotion, through the proliferation and clonal expansion of mutated cells (6). In this process, IBD patient colon can develop anatomic abnormalities such as polyps and colon shortening, therefore routine screening through colonoscopy, is one of the best ways to prevent and monitor the disease. However, recent studies showed that, in contrast to sporadic CRC, CAC evolves from polymorphous non-adenoma-like dysplastic lesions instead of polypoid adenoma, making the detection through colonoscopy much more difficult (5).

The most effective treatment is the complete surgical removal of the lesions and local metastases; unfortunately, despite the extensive colonoscopy screening programs, nearly a quarter of CRCs are diagnosed at an advanced stage, requiring adjuvant chemo-, targeted/immune- or radiation therapy (7,8). In the first case, current guidelines include both a single-agent and multiple-agent therapy, the latter recommended for Stage II and III colon cancers⁷. In fact, fluoropyrimidine (5-FU) is either employed by itself or in combination with oxaliplatin (OX), irinotecan (IRI), leucovorin (LV) and capecitabine (CAP or XELODA or XEL) (7,8,9). On the other hand, adjuvant radiation therapy only seems to be effective in certain high-risk group of CRC, especially if in combination with surgery and chemotherapy (7). Both chemo- and radiotherapy present important side effects such as systemic toxicity, low tumor-specific selectivity, low success rates and high resistance. On the other hand, novel therapies are being developed to target specific dysregulated pathways or mutated proteins/receptors, helping regulate cell proliferation, differentiation and migration and alter the tumor microenvironment (7). Targeted therapies consist mainly in monoclonal antibodies or small molecules such as bevacizumab or aflibercept (anti-VEGF mAb or molecule respectively), cetuximab or panitumumab (anti-EGFR, affecting tyrosine kinase signaling at the surface of the cell membrane), ramucirumab (anti-VEGFR2) or BRAF-inhibitors among others (7,8). Potential challenges regarding the targeted therapies include for example adverse events, cellular bypass mechanisms between pathways leading to acquired resistance and efficacy differences among people (8).

In addition to CRC treatments, a few therapies are currently under investigation for CAC. The first and the most successful ones are 5-aminosalicylic acid and immunomodulators such as thiopurines, which are usually used for the treatment of mild to moderate IBD and have been associated with a reduction in CAC, albeit with some conflicting results (5). In addition to that, the chemopreventive effect of thalidomide is also under investigation. This is due to its therapeutic effect in the treatment of moderate UC and CD, linked to the suppression of macrophage polarization in the tumor microenvironment, reducing inflammation and promoting mucosal healing (10). There are conflicting results in the use of biological agents commonly used for treatment of IBD such as anti-TNFα, JAK inhibitor, IL-12/IL-23 antagonist and anti-integrin α4β7, even though in most cases they appear to have no protective effect against CAC (5). Finally, meta-analysis and cohort studies focused on other IBD treatments such as corticosteroids, folic acid, Non-Aspirin Nonsteroidal Anti-inflammatory Drugs (NA-NSAIDs), calcium supplements and statins showed no statistical effect (5,11). WO2021094620 discloses antibodies against TMEM219 receptor and their use for the treatment of diabetes and intestinal or bowel disorders. However, as mentioned above, most of known treatments for IBD proved ineffective for the treatment of colorectal cancer or provided conflicting results.

In view of the above, it is evident that, despite many technical advances in the field, a molecule for use in the treatment of colorectal cancer, in particular of colitis-associated colorectal cancer, is still needed.

### SUMMARY OF THE INVENTION

It has now been found that an anti-TMEM219 antibody in an AOM/DSS model of colon carcinogenesis significantly reduces tumor density, size and progression.

Therefore, it is an object of the present invention an antibody that binds to TMEM219 receptor or antigen binding fragment thereof for use in the prevention and/or treatment of colorectal cancer.

In an embodiment, said antibody inhibits or reduces the binding of IGFBP3 to said TMEM219 receptor.

Preferably the isolated antibody or antigen binding fragment thereof inhibits, reduces, or neutralizes the activation of the TMEM219 receptor induced by binding of IGFBP3. Activation of the TMEM219 receptor induced by IGFBP3 may be measured by any known method in the art or as described below. In particular, IGFBP3-induced activation of a TMEM219 receptor may be measured by measuring apoptosis increase as described herein or decrease in minigut growth as known in the art and described in several publications (23,24, 25, 26).

Preferably the isolated antibody or antigen binding fragment thereof does not activate TMEM219 pathway upon binding to human TMEM219.

In a preferred embodiment, said antibody or antigen binding fragment thereof comprises:
a. a heavy chain variable domain (VH) comprising:
   i. a CDR1 sequence of the amino acid sequence selected from the group consisting of: SEQ ID NO: 1, 8, 10, 56, 59, 62, 65 and 68;
   ii. a CDR2 sequence of the amino acid sequence selected from the group consisting of: SEQ ID NO: 2, 5, 11, 57, 60, 63, 66 and 69; and
   iii. a CDR3 sequence of the amino acid sequence selected from the group consisting of: SEQ ID NO: 3, 6, 7, 9, 12, 13, 58, 61, 64, 67 and 70; and/or
b. a light chain variable domain (VL) comprising:
   i. a CDR1 sequence of the amino acid sequence selected from the group consisting of: SEQ ID NO:14, 17, 20, 23, 26, 29, 71, 77, 80, 82 and 85;
   ii. a CDR2 sequence of the amino acid sequence selected from the group consisting of: SEQ ID NO: 15, 18, 21, 24, 27, 30, 72, 78, 83 and 86; and
   iii. a CDR3 sequence of the amino acid sequence selected from the group consisting of: SEQ ID NO: 16, 19, 22, 25, 28, 31, 73, 74, 75, 76, 79, 81, 84, 87, 166 and 167.

Preferably the isolated antibody or antigen binding fragment thereof comprises:
- as VHCDR1 SEQ ID NO: 8, as VHCDR2 SEQ ID NO: 5, as VHCDR3 SEQ ID NO: 6, as VLCDR1 SEQ ID NO: 17, as VLCDR2 SEQ ID NO: 18 and as VLCDR3 SEQ ID NO: 19 or Kabat, IMGT, Chothia, AbM, or Contact CDRs of TC01 or of TC05 or
- as VHCDR1 SEQ ID NO: 8, as VHCDR2 SEQ ID NO: 5, as VHCDR3 SEQ ID NO: 6, as VLCDR1 SEQ ID NO: 17, as VLCDR2 SEQ ID NO: 18 and as VLCDR3 SEQ ID NO: 166 or Kabat, IMGT, Chothia, AbM, or Contact CDRs of TC03 or
- as VHCDR1 SEQ ID NO: 8, as VHCDR2 SEQ ID NO: 5, as VHCDR3 SEQ ID NO: 6, as VLCDR1 SEQ ID NO: 17, as VLCDR2 SEQ ID NO: 18 and as VLCDR3 SEQ ID NO: 167 or Kabat, IMGT, Chothia, AbM, or Contact CDRs of TC04 or
- as VHCDR1 SEQ ID NO: 68, as VHCDR2 SEQ ID NO: 69, as VHCDR3 SEQ ID NO: 70, as VLCDR1 SEQ ID NO: 85, as VLCDR2 SEQ ID NO: 86 and as VLCDR3 SEQ ID NO: 87, or Kabat, IMGT, Chothia, AbM, or Contact CDRs of TM1.

Preferably the isolated antibody or antigen binding fragment thereof comprises the CDRs as indicated in any of Tables 1-6.

Preferably the isolated antibody or antigen binding fragment thereof comprises:
a. a heavy chain variable domain sequence of the amino acid sequence selected from the group consisting of: SEQ ID NO:32 to SEQ ID NO:37 or of SEQ ID NO:88 to SEQ ID NO:95; or of SEQ ID NO:168, SEQ ID NO:169 and SEQ ID NO:170; or
b. a light chain variable domain sequence of the amino acid sequence selected from the group consisting of: SEQ ID NO:38 to SEQ ID NO:43; or of SEQ ID NO:96 to SEQ ID NO:103 or of SEQ ID NO:171, SEQ ID NO:172 or SEQ ID NO:173;
c. the heavy chain variable domain of (a) and the light chain variable domain of (b).

Still preferably the isolated antibody is TC01, TC03, TC04, TC05, TA02, TC02, TD01, TE01, TG02, TM1, TE02.1, TE02.2, TE02.3, TE03, TE04, TE07, TE10 or antigen binding fragment thereof, preferably the isolated antibody is TC01, TC05, TC03, TC04 or TM1 or antigen binding fragment thereof, as reported in Tables 1-8. Preferably the isolated antibody is TC01.

Still preferably the isolated antibody is TC01 comprising SEQ ID NO:33 and SEQ ID NO:39, TC03 comprising SEQ ID NO:168 and SEQ ID NO:171, TC04 comprising SEQ ID NO:169 and SEQ ID NO:172, TC05 comprising SEQ ID NO:170 and SEQ ID NO:173, TA02 comprising SEQ ID NO:32 and SEQ ID NO:38, TC01 comprising SEQ ID NO:33 and SEQ ID NO:39, TC02 comprising SEQ ID NO:34 and SEQ ID NO:40, TD01 comprising SEQ ID NO:35 and SEQ ID NO:41, TE01 comprising SEQ ID NO:36 and SEQ ID NO:42, TG02 comprising SEQ ID NO:37 and SEQ ID NO:43, TE02.1 comprising SEQ ID NO:88 and SEQ ID NO:96, TE02.2 comprising SEQ ID NO:89 and SEQ ID NO:97, TE02.3 comprising SEQ ID NO:90 and SEQ ID NO:98, TE03 comprising SEQ ID NO:91 and SEQ ID NO:99, TE04 comprising SEQ ID NO:92 and SEQ ID NO:100, TE07 comprising SEQ ID NO:93 and SEQ ID NO:101, TE10 comprising SEQ ID NO:94 and SEQ ID NO:102, or TM1 comprising SEQ ID NO:95 and SEQ ID NO:103. Preferably the isolated antibody or antigen binding fragment thereof for the use of the invention is a human or humanized antibody.

More preferably it is an IgG2 or IgG4 antibody, preferably an IgG2 kappa antibody, an IgG2 lambda antibody, an IgG4 kappa antibody or an IgG4 lambda antibody, preferably said IgG2 or IgG4 is human IgG2 or human IgG4.

The invention provides an isolated polynucleotide comprising at least one sequence that encodes the antibody or antigen binding fragment thereof as defined above, preferably said polynucleotide is a cDNA, for use for the prevention and/or treatment of colorectal cancer.

The invention provides a vector comprising the polynucleotide as defined above, preferably said vector is selected from the group consisting of a plasmid, a viral vector, a non-episomal mammalian vector, an expression vector, and a recombinant expression vector, for use for the prevention and/or treatment of colorectal cancer.

The invention further provides an isolated cell comprising the polynucleotide as defined above or the vector as defined above, preferably the isolated cell is a hybridoma or a Chinese Hamster Ovary (CHO) cell or a Human Embryonic Kidney cell (HEK293), for use for the prevention and/or treatment of colorectal cancer.

The invention provides also a pharmaceutical composition comprising an antibody that binds to TMEM219, for example as defined above, or the isolated polynucleotide or the vector or the isolated cell as defined above, and at least one pharmaceutically acceptable carrier for use in the prevention and/or treatment of colorectal cancer.

The invention provides a method of prevention and/or treatment of colorectal cancer, the method comprising administering to a subject in need thereof a pharmaceutical composition comprising an antibody that binds to TMEM219, for example as defined above, or the isolated polynucleotide or the vector or the isolated cell as defined above, and at least one pharmaceutically acceptable carrier or administering to a subject in need thereof an antibody that binds to TMEM219 or the isolated polynucleotide or the vector or the isolated cell as defined above.

In a preferred embodiment, said colorectal cancer is colitis-associated colorectal cancer. In an embodiment, the anti-TMEM219 antibody molecule for use for the prevention and/or treatment of colorectal cancer is used in combination with at least one further therapeutic agent.

The additional therapeutic agent may be any active ingredient which is known to be effective in the prevention and/or treatment of cancer, for example it can be a chemotherapeutic agent or a radiotherapy agent. In a preferred embodiment, it is an agent commonly used or useful in the prevention and/or treatment of colorectal cancer. For example it can be selected from fluoropyrimidine (5-FU), oxaliplatin (OX), irinotecan (IRI), leucovorin (LV), capecitabine (CAP or XELODA or XEL), anti-VEGF monoclonal antibodies or small molecules, such as bevacizumab or aflibercept, cetuximab, panitumumab, nimotuzumab, necitumumab (anti-EGFR, affecting tyrosine kinase signaling at the surface of the cell membrane), ramucirumab (anti-VEGFR2), regorafenib (tyrosine kinase inhibitor targeting VEGFR, platelet-derived growth factor, fibroblast growth factor, and BRAF) or BRAF-inhibitors (encorafenib), 5-aminosalicylic acid, immunomodulators such as thiopurines and immune checkpoint inhibitors (pembrolizumab).

### DETAILED DESCRIPTION OF THE INVENTION

### Figures

Figure 1. Schematic representation of the AOM/DSS murine model of colitis-associated cancer.
Figure 2. Schematic representation of the AOM/DSS murine model of colitis-associated cancer, including dosing regimen.
Figure 3. Schematic representation of proximal and distal colon used in this study.
Figure 4. Schematic representation of the AOM/DSS murine model of colitis-associated cancer, including the multistep tumor progression, represented on the timeline and based on the GIN-adenoma-carcinoma sequence. GIN: Gastrointestinal intraepithelial neoplasia; LGA: Low-grade adenoma; HGA: High-grade adenoma; K: Adenocarcinoma. Dark grey dotted lines delineate specific tumoral lesions.
Figure 5. Percentage of body weight loss (A) and Disease Activity Index (DAI) (B) have been quantified at the indicated time points. Values are and mean ± SEM (n=8 per group). The vertical dotted line represents the initiation of Ent001 and anti-TNFalpha administration.
Figure 6. DAI quantified at sacrifice (Day 56). Values are mean ± SEM (n=8 mice/groups). P: **** p<0.0001 vs AOM/DSS+ vehicle and ^^^^ p<0.0001 vs AOM/DSS + Ent001 long-term by one-way ANOVA with multiple comparisons.
Figure 7. Colon length at sacrifice (Day 56). Values are mean ± SEM (n=8 per group). P: **** p<0.0001 vs AOM/DSS+ vehicle; ^^^^ p<0.001 and ^^^ p<0.001 vs AOM/DSS + Ent001 long-term by one-way ANOVA with multiple comparisons.
Figure 8. Graph representing the total histological score of colitis at sacrifice (Day 56) calculated by Rachimilewitz scoring system. Values are mean ± SEM (n=7 per group) vs AOM/DSS+ vehicle.
Figure 9. Graphs represent histological grading of colitis at sacrifice (Day 56). (A) Total histological score (B) Severity of inflammation score (C) Extent of inflammation score (D) Regeneration score, (E) Crypt damage. Values are mean ± SEM (n=7 per group). P: **** p<0.0001 vs AOM/DSS+ vehicle; ^^^^ p<0.001 vs AOM/DSS + Ent001 long-term by one-way ANOVA with multiple comparisons.
Figure 10. Representative H&E-stained images of distal colons from the indicated groups of mice at the end of the experiment (Day 56). M: Mucosa; SM; Submucosa; Mu: Muscular layer or muscularis propria.
Figure 11. Total number of tumors per mouse analyzed at sacrifice (Day 56). Values are mean ± SEM (n=7 per group). P: ****p<0,0001 vs AOM/DSS + Vehicle; ^^ p<0.01 vs AOM/DSS + Ent001 long-term by one-way ANOVA with multiple comparisons.
Figure 12. Number of tumors per mouse divided by tumor grade at sacrifice (Day 56). Values are mean ± SEM (n=7 per group). P: ****p<0.0001 and ** p<0.01 vs AOM/DSS + Vehicle; ^^^ p<0.001 vs AOM/DSS + Ent001 long-term by two-way ANOVA with multiple comparisons. GIN: glandular intraepithelial neoplasia; LGA: Low-grade adenoma; HGA: High-grade adenoma; K: carcinoma.
Figure 13. Tumor size at sacrifice (Day 56). Values are mean ± SEM (n=7 per group). P: ****p<0.0001 and **p<0.01 vs AOM/DSS + Vehicle and ^ p<0.05 vs AOM/DSS + Ent001 long-term by two-way ANOVA with multiple comparisons. LGA: Low-grade adenoma; HGA: High-grade adenoma; K: carcinoma.
Figure 14. Representative H&E images of colons showing different LGA, HGA and K sizes in the indicated groups. Dotted red lines delineate tumors. Scale bar: 100 µm.
Figure 15. Number of Brdu+ nuclei/mm² in inflammatory areas at sacrifice (Day 56). Values are mean ± SEM (n=4 per group). P: ****<0.0001 vs AOM/DSS + Vehicle by one-way ANOVA with multiple comparisons.
Figure 16. Representative BrdU-stained images of colons showing BrdU-positive epithelial cells in inflammatory areas of AOM/DSS-treated mice in the indicated groups. Scale bar: 100 µm.
Figure 17. Number of BrdU-positive nuclei/LGA area in AOM/DSS-treated mice at sacrifice (Day 56). Values are mean ± SEM (n=4 per group except for the Ent001 long-term group in which only 3 mice displayed LGA). P: **p<0.01 vs AOM/DSS + Vehicle by one-way ANOVA with multiple comparisons.
Figure 18. Representative BrdU-stained images of colons showing BrdU-positive epithelial cells in LGA tumors, in the indicated groups.
Figure 19. Ratio of Cleaved Caspase 8 over total Caspase 8 in colon lysate at sacrifice (Day 56). Values in the upper panel were obtained by densitometric analysis using Image J (pool of n=4 colon lysates). The ratio between cleaved and total Caspase 8 has been normalized over actin, as loading control.

The inventors found TMEM219 as a therapeutic target for the treatment of colorectal cancer, in particular colitis-associated colorectal cancer.

Accordingly, an anti-TMEM219 antibody can be advantageously used for the prevention and/or treatment of colorectal cancer.

For "colorectal cancer" or "CRC" is intended a cancer which begins in and/or affects at least a portion of the colon and/or of the rectum. The cancer can be at any stage, for example at stage 0, stage I, stage II, stage III or stage IV according to classifications known in the field. Colorectal cancer comprises colon cancer, bowel cancer, and rectal cancer. The cancer can also be a metastasis of another cancer. In an embodiment, the cancer is colitis-associated colorectal cancer. For "colitis-associated colorectal cancer" or "CAC" it is intended a type of colorectal cancer which is preceded by clinically detectable inflammatory bowel disease (IBD), such as Crohn's disease (CD) or Ulcerative colitis.

"Tumor" and "cancer" are herein used as synonyms.

For "prevention" is intended that administration of the agent decreases the chance of developing a disease or condition, i.e. it decreases the chance of developing a cancer, in particular a colorectal cancer. In some embodiments, for "prevention" is intended that administration of the agent stops or slows down progression of a disease that has already begun. For example, in some embodiments the agent of the invention is administered to a subject who already has cancer and the cancer does not develop to a more advanced stage.

For "treatment" is intended that administration of the agent improves or cures or reverts a condition or a disease, i.e. it improves or cures or reverts a cancer. In some embodiments, for "treatment" it is intended that the disease, such as cancer, is not completely cured but it reverts to a less advanced stage.

Preferably, colorectal cancer is selected from the group consisting of colon cancer, bowel cancer, and rectal cancer.

Preferably, colorectal cancer is colitis-associated colorectal cancer.

For "antibody that binds to TMEM219" is intended an antibody able to bind and/or block and/or neutralize, at least partially, TMEM219 receptor. Preferably it is an antibody that specifically binds TMEM219.

TMEM219 is a known receptor of IGFBP3, an insulin-like growth factor binding protein. Anti-TMEM219 antibody or anti-TMEM antibody or antibody against TMEM219 or antibody that binds to TMEM219 are herein used as synonyms.

By "antibody that specifically binds" TMEM219 is intended that the antibody will not substantially cross react with another, nonhomologous, human polypeptide. By "not substantially cross react" is intended that the antibody or fragment has a binding affinity for a non-homologous protein which is less than 10%, more preferably less than 5%, and even more preferably less than 1%, of the binding affinity for TMEM219.

In various embodiments, an antibody that "specifically binds" TMEM219, as used herein, includes antibodies that bind TMEM219 or the extracellular portion thereof, such as ecto-TMEM219, with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or about 0.5 nM, as measured with an Octet biolayer interferometry device or in a surface plasmon resonance assay, for example using the BIAcore^{™} system (Biacore Life Sciences division of GE Healthcare, Piscataway, NJ) or kinetic exclusion assays or any known method in the art.

The term "antibody" herein is used in the broadest sense understood in the art, including all polypeptides described as antibodies.

For example, the term "antibody", as used herein, encompasses monoclonal antibodies, polyclonal antibodies, monospecific and multispecific antibodies (e.g., bispecific antibodies), and antibody fragments, so long as the fragment exhibits the desired antigen-binding activity (antigen-binding fragments). The term has its broadest art-recognized meaning and includes all known formats, including, without limitation: bivalent monospecific monoclonal antibodies, bivalent bispecific antibodies, trivalent trispecific antibodies, F(ab) fragments, F(ab)'2 fragments, scFv fragments, diabodies, single domain antibodies, including camelid VHH single domain antibodies, tandAbs, and flexibodies.

The terms "antigen-binding fragment" of an antibody or equivalently "antigen-binding portion" of an antibody and the like, as used herein, include any naturally occurring, enzymatically obtainable, synthetic, or genetically engineered polypeptide or glycoprotein that comprises a portion of an antibody and that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

As with full antibody molecules, antigen-binding fragments may be monospecific or multispecific (e.g., bispecific). A multispecific antigen-binding fragment of an antibody will typically comprise at least two different variable domains, wherein each variable domain is capable of specifically binding to a separate antigen or to a different epitope on the same antigen.

In particular embodiments, an antigen-binding fragment of an antibody comprises at least one variable domain covalently linked to at least one constant domain. Non-limiting, exemplary configurations of variable and constant domains that may be found within an antigen-binding fragment of an antibody include: (i) VH- CH1; (ii) VH-CH2; (iii) VH-CH3; (iv) VH-CH1-CH2; (v) VH-CH1-CH2-CH3; (vi) VH- CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may in various embodiments consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody may in various embodiments comprise a homo-dimer or hetero-dimer (or other multimer) of any of the variable and constant domain configurations listed above in non-covalent association with one another and/or with one or more monomeric VH or VL domain (e.g., by disulfide bond(s)).

The term "antigen-binding fragment" of an antibody further includes single domain antibodies.

A single-domain antibody is an antibody fragment consisting of a single monomeric variable antibody domain. In some embodiments, the single-domain antibody is derived from the variable domain of the antibody heavy chain from camelids (also termed nanobodies, or VHH fragments). In some embodiments, the single-domain antibody is an autonomous human heavy chain variable domain (aVH) or VNAR fragments derived from sharks.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain- deleted antibodies, chimeric antibodies, CDR-grafted antibodies, diabodies, triabodies, tetrabodies, minibodies, nanobodies (e.g., monovalent nanobodies, and bivalent nanobodies), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL or VL-VL dimers. Alternatively, the antigen-binding fragment of an antibody may contain a monomeric VH or VL domain.

The antibody or binding molecule of the invention can further be linked to an active substance, preferably a nanoparticle or a radionucleotide.

As used herein, the term "antigen binding molecule" refers in its broadest sense to a molecule that specifically binds an antigenic determinant. Examples of antigen binding molecules are antibodies, including antigen-binding antibody fragments, and scaffold antigen binding proteins.

The term "antigen binding moiety" refers to the portion of an antigen binding molecule that specifically binds to an antigenic determinant. Antigen binding moieties include antibodies and antigen-binding fragments thereof, such as scFv, that are capable of specific binding to an antigen on a target cell. In a particular aspect, the antigen binding moiety is able to direct the entity to which it is attached, such as a cell, to a target site. Anti-TMEM219 antibodies are known in the field. For example, anti-TMEM219 antibodies are described in WO2021094620.

Preferred anti-TMEM219 antibodies for the uses of the invention are as defined above and as disclosed in WO2021094620. Particularly preferred antibodies are TC01, TC03, TC04, TC05, TA02, TC02, TD01, TE01, TG02, TM1, TE02.1, TE02.2, TE02.3, TE03, TE04, TE07, TE10 as disclosed in WO2021094620 and herein in Tables 1-8, or encoded by the nucleotide sequences in Tables 9-10. A preferred anti-TMEM219 antibody is TC01, also herein named as Ent001.

In a preferred embodiment, said anti-TMEM219 antibody for the use of the invention is TC01 and it comprises a light chain comprising a light variable region comprising or consisting of a sequence of SEQ ID NO. 39 and a light constant region comprising or consisting of a sequence of SEQ ID NO. 123 and/or a heavy chain comprising a heavy variable region comprising or consisting of a sequence of SEQ ID NO. 33 and a heavy constant region comprising or consisting of a sequence of SEQ ID NO. 120 or of SEQ ID NO. 180.

In a particular embodiment, said anti-TMEM219 antibody is TC01 and it comprises at least:
a light chain comprising or consisting of the following sequence: 
QAVLTQPPSVSVSPGQTASITCSGDKLGNKNAYWYQQKPGQSPVLVMYQSTRRPS
GIPERFSASNSGNTATLTISGTQAMDEADYYCQAWDSSSGWEVFGGGTKLTVLGQP
KAAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS (SEQ ID NO. 4)
 and/or
an heavy chain comprising or consisting of the following sequence: 
QIQLVQSGAEVKKPGASVKVSCKASGYTFTSYGISWVRQAPGQGLEWMGWISAYNG
NTNYAQKLQGRVTMTTDTSTSTAYMELRSLRSDDTAVYYCARWGRWLAHDYWGQG
TLVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCP
PCPAPEFEGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISKAKG
QPREPQVYTLPPSQEEMTKNQVSLTCLVKGFYPSDIAVEWESNGQPENNYKTTPPVL
DSDGSFFLYSRLTVDKSRWQEGNVFSCSVMHEALHNHYTQKSLSLSLGK (SEQ ID
NO. 125)
or of the following sequence:

QIQLVQSGAEVKKPGASVKVSCKASGYTFTSYGISWVRQAPGQGLEWMGWISAYNG
NTNYAQKLQGRVTMTTDTSTSTAYMELRSLRSDDTAVYYCARWGRWLAHDYWGQG
TLVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCP
PCPAPEFEGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISKAKG
QPREPQVYTLPPSQEEMTKNQVSLTCLVKGFYPSDIAVEWESNGQPENNYKTTPPVL
DSDGSFFLYSRLTVDKSRWQEGNVFSCSVMHEALHNHYTQKSLSLSLG (SEQ ID
NO. 126)

In an embodiment, the anti-TMEM219 antibody molecule comprises at least one antigen-binding region, e.g., a variable region or an antigen-binding fragment thereof, from an antibody described herein, e.g., an antibody chosen from any of TA02, TC01, TC02, TD01, TE01, TG02, TM1, TE02.1, TE02.2, TE02.3, TE03, TE04, TE07, TE10 as defined in Tables 1-8 or encoded by the nucleotide sequences in Tables 9-10; or a sequence substantially identical (e.g., at least 80%, 85%, 90%, 92%, 95%, 97%, 98%, 99% or higher identical) to any of the aforesaid sequences.

In yet another embodiment, the anti-TMEM219 antibody molecule comprises at least one, two, three or four variable regions from an antibody described herein, e.g., an antibody chosen from any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., at least 80%, 85%, 90%, 92%, 95%, 97%, 98%, 99% or higher identical) to any of the aforesaid sequences.

In yet another embodiment, the anti-TMEM219 antibody molecule comprises at least one or two heavy chain variable regions from an antibody described herein, e.g., an antibody chosen from any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., at least 80%, 85%, 90%, 92%, 95%, 97%, 98%, 99% or higher identical) to any of the aforesaid sequences.

In yet another embodiment, the anti-TMEM219 antibody molecule comprises at least one or two light chain variable regions from an antibody described herein, e.g., an antibody chosen from any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., at least 80%, 85%, 90%, 92%, 95%, 97%, 98%, 99% or higher identical) to any of the aforesaid sequences.

In yet another embodiment, the anti-TMEM219 antibody molecule includes a heavy chain constant region for an IgG4, e.g., a human IgG4. In one embodiment, the human IgG4 includes a substitution at position 228 (e.g., a Ser to Pro substitution). In one embodiment, the human IgG4 includes a substitution at position 235 (e.g., a Leu to Glu substitution). In one embodiment, the human IgG4 includes a substitution at position 228 (e.g., a Ser to Pro substitution) and a substitution at position 235 (e.g., a Leu to Glu substitution). In still another embodiment, the anti-TMEM219 antibody molecule includes a heavy chain constant region for an IgG1, e.g., a human IgG1. In one embodiment, the human IgG1 includes a substitution at position 297 (e.g., an Asn to Ala substitution). In one embodiment the human IgG1 includes a substitution at position 250, a substitution at position 428, or both (e.g., a Thr to Gln substitution at position 250 and/or a Met to Leu substitution at position 428). In one embodiment, the human IgG1 includes a substitution at position 234, a substitution at position 235, or both (e.g., a Leu to Ala substitution at position 234 and/or a Leu to Ala substitution at position 235).

In yet another embodiment, the anti-TMEM219 antibody molecule includes a kappa light chain constant region, e.g., a human kappa light chain constant region. In one embodiment, the light chain constant region comprises an amino sequence set forth in Table 11, or a sequence substantially identical (e.g., at least 80%, 85%, 90%, 92%, 95%, 97%, 98%, 99% or higher identical) thereto.

In another embodiment, the anti-TMEM219 antibody molecule includes a heavy chain constant region for an IgG4, e.g., a human IgG4, and a kappa light chain constant region, e.g., a human kappa light chain constant region. In one embodiment, the human IgG1 or IgG4 includes a substitution at the variable region to decrease aggregation, reduce charge heterogeneity, increase affinity and modulate antigen binding; removal by mutation of instability hotspot in the CDR, putative N-glycosylation sites in the variable region as described in (27), incorporated by reference. In one embodiment, the heavy chain constant region comprises an amino sequence set forth in Table 11, or a sequence substantially identical (e.g., at least 80%, 85%, 90%, 92%, 95%, 97%, 98%, 99% or higher identical) thereto.

In another embodiment, the anti-TMEM219 antibody molecule includes a heavy chain variable domain and a constant region, a light chain variable domain and a constant region, or both, comprising the amino acid sequence of any of TA02, TC01, TC02, TD01, TE01, TG02, TM1, TE02.1, TE02.2, TE02.3, TE03, TE04, TE07, TE10 as defined in Tables 1-8, 11 or encoded by the nucleotide sequences in Tables 9-10; or a sequence substantially identical (e.g., at least 80%, 85%, 90%, 92%, 95%, 97%, 98%, 99% or higher identical) to any of the aforesaid sequences. The anti-TMEM219 antibody molecule, optionally, comprises a leader sequence from a heavy chain, a light chain, or both.

In yet another embodiment, the anti-TMEM219 antibody molecule includes at least one, two, or three complementarity determining regions (CDRs) from a heavy chain variable region of an antibody described herein, e.g., an antibody chosen from any of any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., at least 80%, 85%, 90%, 92%, 95%, 97%, 98%, 99% or higher identical) to any of the aforesaid sequences.

In yet another embodiment, the anti-TMEM219 antibody molecule includes at least one, two, or three CDRs (or collectively all of the CDRs) from a heavy chain variable region comprising an amino acid sequence shown in Tables 1-8 or encoded by a nucleotide sequence shown in Tables 9-10. In one embodiment, one or more of the CDRs (or collectively all of the CDRs) have one, two, three, four, five, six or more changes, e.g., amino acid substitutions or deletions, relative to the amino acid sequence shown in Tables 1-8 or encoded by a nucleotide sequence shown in Tables 9-10.

In yet another embodiment, the anti-TMEM219 antibody molecule includes at least one, two, or three CDRs from a light chain variable region of an antibody described herein, e.g., an antibody chosen from any of any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., at least 80%, 85%, 90%, 92%, 95%, 97%, 98%, 99% or higher identical) to any of the aforesaid sequence.

In yet another embodiment, the anti-TMEM219 antibody molecule includes at least one, two, or three CDRs (or collectively all of the CDRs) from a light chain variable region comprising an amino acid sequence shown in Tables 1-8 or encoded by a nucleotide sequence shown in Tables 9-10. In one embodiment, one or more of the CDRs (or collectively all of the CDRs) have one, two, three, four, five, six or more changes, e.g., amino acid substitutions or deletions, relative to the amino acid sequence shown in Tables 1-8, or encoded by a nucleotide sequence shown in Tables 9-10. In certain embodiments, the anti-TMEM219 antibody molecule includes a substitution in a light chain CDR, e.g., one or more substitutions in a CDR1, CDR2 and/or CDR3 of the light chain.

In another embodiment, the anti-TMEM219 antibody molecule includes at least one, two, three, four, five or six CDRs (or collectively all of the CDRs) from a heavy and light chain variable region comprising an amino acid sequence shown in Tables 1-8 or encoded by a nucleotide sequence shown in Tables 9-10. In one embodiment, one or more of the CDRs (or collectively all of the CDRs) have one, two, three, four, five, six or more changes, e.g., amino acid substitutions or deletions, relative to the amino acid sequence shown in Tables 1-8, or encoded by a nucleotide sequence shown in Tables 9-10.

In one embodiment, the anti-TMEM219 antibody molecule includes all six CDRs from an antibody described herein, e.g., an antibody chosen from any of any of TA02, TC01, TC02, TD01, TE01, TG02, TM1, TE02.1, TE02.2, TE02.3, TE03, TE04, TE07, TE10 as defined in Tables 1-8, or encoded by the nucleotide sequences in Tables 9-10, or closely related CDRs, e.g., CDRs which are identical or which have at least one amino acid alteration, but not more than two, three or four alterations (e.g., substitutions, deletions, or insertions, e.g., conservative substitutions).

In one embodiment, the anti-TMEM219 antibody molecule may include any CDR described herein. In certain embodiments, the anti-TMEM219 antibody molecule includes a substitution in a light chain CDR, e.g., one or more substitutions in a CDR1, CDR2 and/or CDR3 of the light chain. In another embodiment, the anti-TMEM219 antibody molecule includes at least one, two, or three CDRs according to Kabat et al. (e.g., at least one, two, or three CDRs according to the Kabat definition as set out in Tables 3, 5, 6) from a heavy chain variable region of an antibody described herein, e.g., an antibody chosen from any of any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 3, 5, 6.

In one embodiment, the anti-TMEM219 antibody molecule comprises:
(i) a heavy chain variable region (VH) comprising a VHCDR1 amino acid sequence chosen from any one of SEQ ID NO: 1, 8, 10, 56, 59, 62, 65 and 68; a VHCDR2 amino acid sequence chosen from any one of SEQ ID NO: 2, 5, 11, 57, 60, 63, 66 and 69; and a VHCDR3 amino acid sequence chosen from any one of SEQ ID NO: 3, 6, 7, 9, 12, 13, 58, 61, 64, 67 and 70; and
(ii) a light chain variable region (VL) comprising a VLCDR1 amino acid sequence chosen from any one of SEQ ID NO: 14, 17, 20, 23, 26, 29, 71, 77, 80, 82 and 85, a VLCDR2 amino acid sequence chosen from any one of SEQ ID NO: 15, 18, 21, 24, 27, 30, 72, 78, 83 and 86, and a VLCDR3 amino acid sequence chosen from SEQ ID NO: 16, 19, 22, 25, 28, 31, 73, 74, 75, 76, 79, 81, 84 and 87.

In one embodiment, the light or the heavy chain variable framework (e.g., the region encompassing at least FR1, FR2, FR3, and optionally FR4) of the anti-TMEM219 antibody molecule can be chosen from: (a) a light or heavy chain variable framework including at least 80%, 85%, 87% 90%, 92%, 93%, 95%, 97%, 98%, or preferably 100% of the amino acid residues from a human light or heavy chain variable framework, e.g., a light or heavy chain variable framework residue from a human mature antibody, a human germline sequence, or a human consensus sequence; (b) a light or heavy chain variable framework including from 20% to 80%, 40% to 60%, 60% to 90%, or 70% to 95% of the amino acid residues from a human light or heavy chain variable framework, e.g., a light or heavy chain variable framework residue from a human mature antibody, a human germline sequence, or a human consensus sequence; (c) a non-human framework (e.g., a rodent framework); or (d) a non-human framework that has been modified, e.g., to remove antigenic or cytotoxic determinants, e.g., deimmunized, or partially humanized. In one embodiment, the light or heavy chain variable framework region (particularly FR1, FR2 and/or FR3) includes a light or heavy chain variable framework sequence at least 70, 75, 80, 85, 87, 88, 90, 92, 94, 95, 96, 97, 98, 99% identical or identical to the frameworks of a VL or VH segment of a human germline gene.

In certain embodiments, the anti-TMEM219 antibody molecule comprises a heavy chain variable domain having at least one, two, three, four, five, six, seven, ten, fifteen, twenty or more changes, e.g., amino acid substitutions or deletions.

In one embodiment, the heavy or light chain variable region, or both, of the anti-TMEM219 antibody molecule includes an amino acid sequence encoded by a nucleic acid sequence described herein or a nucleic acid that hybridizes to a nucleic acid sequence described herein (e.g., a nucleic acid sequence as shown in Tables 9-10) or its complement, e.g., under low stringency, medium stringency, or high stringency, or other hybridization conditions described herein.

In another embodiment, the anti-TMEM219 antibody molecule comprises at least one, two, three, or four antigen-binding regions, e.g., variable regions, having an amino acid sequence as set forth in Tables 7-8 or a sequence substantially identical thereto, e.g., a sequence at least about 85%, 90%, 95%, 99% or more identical thereto, or which differs by no more than 1, 2, 5, 10, or 15 amino acid residues from the sequences shown in Tables 7-8. In another embodiment, the anti-TMEM219 antibody molecule includes a VH and/or a VL domain encoded by a nucleic acid having a nucleotide sequence as set forth in Tables 9-10 or a sequence substantially identical thereto, e.g., a sequence at least about 85%, 90%, 95%, 99% or more identical thereto, or which differs by no more than 3, 6, 15, 30, or 45 nucleotides from the sequences shown in Tables 9-10.

In yet other embodiments, the anti-TMEM219 antibody molecule has a heavy chain constant region (Fc) chosen from, e.g., the heavy chain constant regions of IgG1, IgG2, IgG3, IgG4, IgM, IgA1, IgA2, IgD, and IgE; particularly, chosen from, e.g., the heavy chain constant regions of IgG1, IgG2, IgG3, and IgG4, more particularly, the heavy chain constant region of IgG1 or IgG4 (e.g., human IgG1, IgG2 or IgG4). In one embodiment, the heavy chain constant region is human IgG1. In another embodiment, the anti-TMEM219 antibody molecule has a light chain constant region chosen from, e.g., the light chain constant regions of kappa or lambda. In one embodiment, the constant region is altered, e.g., mutated, to modify the properties of the anti-TMEM219 antibody molecule (e.g., to increase or decrease one or more of: Fc receptor binding, antibody glycosylation, the number of cysteine residues, effector cell function, complement function, half-life, aggregation and stability). In certain embodiments, the anti-TMEM antibody molecules comprise a human IgG4 mutated. The constant region of an antibody is important in the ability of an antibody to fix complement and mediate cell-dependent cytotoxicity. Thus, the isotype of an antibody may be selected on the basis of whether it is desirable for the antibody to mediate cytotoxicity. In certain embodiments, the constant region is an IgG1, IgG2, IgG3, IgG4 constant region.

In one embodiment, the anti-TMEM219 antibody molecule is isolated or recombinant. In one embodiment, the anti-TMEM219 antibody molecule is a humanized or human antibody molecule.

The term "human antibody", as used herein, is intended to include antibodies having variable and constant regions derived from human germline immunoglobulin sequences. The human antibodies may in various embodiments nonetheless include amino acid residues not encoded by human germline immunoglobulin sequences (e.g., mutations introduced by random or site-specific mutagenesis in vitro or by somatic mutation in vivo), for example in the CDRs, and in some embodiments, CDR3. However, the term "human antibody", as used herein, is not intended to include antibodies in which CDR sequences are derived from the germline of another mammalian species, such as a mouse, which have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res.20:6287-6295, incorporated herein by reference in its entirety,) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody." In various embodiments, the isolated antibody also includes an antibody *in situ* within a recombinant cell. In other embodiments, isolated antibodies are antibodies that have been subjected to at least one purification or isolation step. In various embodiments, an isolated antibody may be substantially free of other cellular material and/or chemicals.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstances, an epitope may include moieties of saccharides, phosphoryl groups, or sulfonyl groups on the antigen.

The invention also features a nucleic acid molecule that comprises one or both nucleotide sequences that encode heavy and light chain variable regions, CDRs, hypervariable loops, framework regions of the anti-TMEM219 antibody molecules, as described herein, for use for the prevention and/or treatment of colorectal cancer. In certain embodiments, the nucleotide sequence that encodes the anti-TMEM219 antibody molecule is codon optimized. For example, a first and second nucleic acids encoding heavy and light chain variable regions, respectively, of an anti-TMEM219 antibody molecule chosen from one or more of, e.g., any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10, or a sequence substantially identical thereto can be used. For example, the nucleic acid can comprise a nucleotide sequence as set forth in Tables 9-10, or a sequence substantially identical thereto (e.g., a sequence at least about 85%, 90%, 95%, 99% or more identical thereto, or which differs by no more than 3, 6, 15, 30, or 45 nucleotides from the sequences shown in Tables 9-10).

In other embodiments, the nucleic acid molecule comprises a nucleotide sequence that encodes a heavy chain variable domain and/or a heavy chain constant region comprising the amino acid sequence of any of TA02, TC01, TC02, TD01, TE01, TG02, TM1, TE02.1, TE02.2, TE02.3, TE03, TE04, TE07, TE10 as defined in Tables 1-8 or encoded by the nucleotide sequence in Tables 9-10; or a sequence substantially identical (e.g., a sequence at least about 85%, 90%, 95%, 99% or more identical) to any of the aforesaid sequences.

In other embodiments, the nucleic acid molecule comprises a nucleotide sequence that encodes a light chain variable domain and/or a light chain constant region comprising the amino acid sequence of any of TA02, TC01, TC02, TD01, TE01, TG02, TM1, TE02.1, TE02.2, TE02.3, TE03, TE04, TE07, TE10 as defined in Tables 1-8 or the nucleotide sequence in Tables 9-10, or a sequence substantially identical (e.g., a sequence at least about 85%, 90%, 95%, 99% or more identical) to any of the aforesaid sequences.

The aforesaid nucleotide sequences encoding the anti-TMEM219 heavy and light chain variable domain and constant regions can be present in a separate nucleic acid molecule, or in the same nucleic acid molecule. In certain embodiments, the nucleic acid molecules comprise a nucleotide sequence encoding a leader sequence.

In certain embodiments, the nucleic acid molecule comprises a nucleotide sequence encoding at least one, two, or three CDRs, or hypervariable loops, from a heavy chain variable region having an amino acid sequence as set forth in Tables 1-8, or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule comprises a nucleotide sequence encoding at least one, two, or three CDRs, or hypervariable loops, from a light chain variable region having an amino acid sequence as set forth in Tables 1-8 or a sequence substantially homologous thereto (e.g., a sequence at least about 85%, 90%, 95%, 99% or more identical thereto, and/or having one, two, three or more substitutions, insertions or deletions, e.g., conserved substitutions).

In yet another embodiment, the nucleic acid molecule comprises a nucleotide sequence encoding at least one, two, three, four, five, or six CDRs, or hypervariable loops, from heavy and light chain variable regions having an amino acid sequence as set forth in Tables 1-8 or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule includes one or more heavy chain framework region (e.g., any of VHFW1 (type a), VHFW1 (type b), VHFW1 (type c), VHFW1 (type d), VHFW2 (type a), VHFW2 (type a'), VHFW2 (type b), VHFW2 (type c), VHFW2 (type d), VHFW2 (type e), VHFW3 (type a), VHFW3 (type b), VHFW3 (type c), VHFW3 (type d), VHFW3 (type e), or VHFW4, or any combination thereof, e.g., a framework combination as described herein) for any of TA02, TC01, TC02, TD01, TE01, TG02, TM1, TE02.1, TE02.2, TE02.3, TE03, TE04, TE07, TE10 as defined in Tables 1-8, or a sequence substantially identical thereto. For example, the nucleic acid molecule can comprise a nucleotide sequence as set forth in Tables 9-10, or a sequence substantially identical thereto (e.g., a sequence at least about 85%, 90%, 95%, 99% or more identical thereto, or which differs by no more than 3, 6, 15, 30, or 45 nucleotides from the sequences shown in Tables 9-10).

In another embodiment, the nucleic acid molecule includes one or more light chain framework region (e.g., any of VLFW1 (type a), VLFW1 (type b), VLFW1 (type c), VLFW1 (type d), VLFW1 (type e), VLFW1 (type f), VLFW2 (type a), VLFW2 (type c), VLFW3 (type a), VLFW3 (type b), VLFW3 (type c), VLFW3 (type d), VLFW3 (type e), VLFW3 (type f), VLFW3 (type g), or VLFW4, or any combination thereof, e.g., a framework combination as described herein) for of any of E01, E02, E08, E14, E19, E20, E23, E24 or M1 as defined in Tables 1-8, or a sequence substantially identical thereto. For example, the nucleic acid molecule can comprise a nucleotide sequence as set forth in Tables 9-10, or a sequence substantially identical thereto (e.g., a sequence at least about 85%, 90%, 95%, 99% or more identical thereto, or which differs by no more than 3, 6, 15, 30, or 45 nucleotides from the sequences shown in Tables 9-10).

In another embodiment, the nucleic acid molecule includes one or more heavy chain framework region and one or more light chain framework region as described herein. The heavy and light chain framework regions may be present in the same vector or separate vectors.

Host cells and vectors containing the nucleic acids described herein or modified for codon optimization according to known methods can also be used. The nucleic acids may be present in a single vector or separate vectors present in the same host cell or separate host cell. The host cell can be a eukaryotic cell, e.g., a mammalian cell, an insect cell, a yeast cell, or a prokaryotic cell, e.g., *E. coli.* For example, the mammalian cell can be a cultured cell or a cell line. Exemplary mammalian cells include lymphocytic cell lines (e.g., NSO), Chinese hamster ovary cells (CHO), COS cells, oocyte cells, and cells from a transgenic animal, e.g., mammary epithelial cell.

In another aspect, the invention provides, compositions, e.g., pharmaceutical compositions, for use for the prevention and/or treatment of colorectal cancer which include a pharmaceutically acceptable carrier, excipient or stabilizer, and at least one anti-TMEM219 antibody, such as the ones described herein. Preferably said pharmaceutical composition includes anti-TMEM219 antibody TC01, as described herein. In one embodiment, the composition, e.g., the pharmaceutical composition, includes a combination of the antibody molecule and one or more agents, e.g., an anticancer therapeutic agent or other anti-TMEM219 antibody molecule, as described herein. In one embodiment, the antibody molecule is conjugated to a label or a therapeutic agent.

The present invention also includes anti-TMEM219 antibodies for the uses of the invention comprising variants of any of the HCVR, LCVR, and/or CDR amino acid sequences disclosed herein having one or more conservative substitutions.

The antibody can be administered to the subject in various embodiments in a formulation comprising suitable carriers, excipients, and other agents to provide improved transfer, delivery, tolerance, and the like, and suitable for an intravenous or subcutaneous injection.

The injectable preparations may be prepared by methods publicly known. For example, injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 20 or 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injectable preparation thus prepared can be filled in an appropriate ampoule.

The antibody for the use of the invention can be administered to the subject using any acceptable device or mechanism. For example, the administration can be accomplished using a syringe and needle or with a reusable pen and/or autoinjector delivery device. The methods of the present invention include the use of numerous reusable pen and/or autoinjector delivery devices to administer an antibody (or pharmaceutical formulation comprising the antibody). Examples of such devices include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen and/or autoinjector delivery devices having applications in subcutaneous delivery of a pharmaceutical composition of the present invention include, but are not limited to, the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), the HUMIRA^{™} Pen (Abbott Labs, Abbott Park, IL), the DAI^{®} Auto Injector (SHL Group) and any auto-injector featuring the PUSHCLICK^{™} technology (SHL Group), to name only a few.

In one embodiment, the antibody is administered with a prefilled syringe. In another embodiment, the antibody is administered with a prefilled syringe containing a safety system. For example, the safety system prevents an accidental needlestick injury. In various embodiments, the antibody is administered with a prefilled syringe containing an ÈRIS^{™} safety system (West Pharmaceutical Services Inc.). See also U.S. patent numbers 5,215,534 and 9,248,242, incorporated herein by reference in their entireties. In another embodiment, the antibody is administered with an auto-injector. In various embodiments, the antibody is administered with an auto-injector featuring the PUSHCLICK^{™} technology (SHL Group). In various embodiments, the auto-injector is a device comprising a syringe that allows for administration of a dose of the composition and/or antibody to a subject. See also U.S. patent numbers 9,427,531 and 9,566,395 3.

According to the invention, "subject" means a human subject or human patient. In one embodiment, the subject is in need of inhibiting, reducing, neutralizing or blocking colorectal cancer. In one embodiment, the subject has, or is at risk of having, colorectal cancer.

The content of the antibody or antigen binding fragment thereof in the pharmaceutical composition for the use of the invention is not limited as far as it is useful for treatment or prevention of colorectal cancer, but preferably contains 0.0000001-10% by weight per total composition. The choice of the carrier may depend upon the route of administration and concentration of the active agent(s) and the carrier may be in the form of a lyophilised composition or an aqueous solution. Generally, an appropriate amount of a pharmaceutically acceptable salt is used in the carrier to render the composition isotonic. Examples of the carrier include but are not limited to saline, Ringer's solution and dextrose solution. Preferably, acceptable excipients, carriers, or stabilizers are non-toxic at the dosages and concentrations employed, including buffers such as citrate, phosphate, and other organic acids; salt-forming counter- ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). Suitable carriers and their formulations are described in greater detail in Remington's Pharmaceutical Sciences, 17th ed., 1985, Mack Publishing Co. The composition may also contain at least one further active compound, such as a chemotherapeutic agent. Preferably, the antibody or antigen binding fragment thereof is included in a pharmaceutical composition for the use of the invention in an effective amount. The term "effective amount" refers to an amount sufficient to induce a detectable therapeutic response in the subject to which the pharmaceutical composition is to be administered.

### SEQUENCES

| **Table 1: VH CDR Sequences** | | | |
|---|---|---|---|
| **Antibody** | **CDR1** | **CDR2** | **CDR3** |
| TA02 | SYAIS (SEQ ID NO. 1) | GIIPIFGTANYAQKF QG (SEQ ID NO. 2) | GDIAAAGRKGLPIY YMDV (SEQ ID NO. 3) |
| TC01 | SYGIS (SEQ ID NO. 8) | WISAYNGNTNYAQ KLQG (SEQ ID NO. 5) | WGRWLAHDY (SEQ ID NO. 6) |
| TC02 | SYAIS (SEQ ID NO. 1) | GIIPIFGTANYAQKF QG (SEQ ID NO. 2) | PSGYYIYDAFDI (SEQ ID NO. 7) |
| TD01 | SYGIS (SEQ ID NO. 8) | WISAYNGNTNYAQ KLQG (SEQ ID NO. 5) | DLGWPDDY (SEQ ID NO. 9) |
| TE01 | DYGMS (SEQ ID NO. 10) | GINWNGGSTGYAD SVKG (SEQ ID NO. 11) | DRLRYCSSTSCYIP DY (SEQ ID NO. 12) |
| TG02 | SYGIS (SEQ ID NO. 8) | WISAYNGNTNYAQ KLQG (SEQ ID NO. 5) | VGSTYDFWSGAYY YYGMDV (SEQ ID NO. 13) |
| TC03 | SYGIS (SEQ ID NO. 8) | WISAYNGNTNYAQ KLQG (SEQ ID NO. 5) | WGRWLAHDY (SEQ ID NO. 6) |
| TC04 | SYGIS (SEQ ID NO. 8) | WISAYNGNTNYAQ KLQG (SEQ ID NO. 5) | WGRWLAHDY (SEQ ID NO. 6) |
| TC05 | SYGIS (SEQ ID NO. 8) | WISAYNGNTNYAQ KLQG (SEQ ID NO. 5) | WGRWLAHDY (SEQ ID NO. 6) |
| TE02.1 | GFTFSRHG (SEQ ID NO. 56) | IWYDGRNK (SEQ ID NO. 57) | AREGITMVRGVIPL FDY (SEQ ID NO. 58) |
| TE02.2 | GFTFSRHG (SEQ ID NO. 56) | IWYDGRNK (SEQ ID NO. 57) | AREGITMVRGVIPL FDY (SEQ ID NO. 58) |
| TE02.3 | GFTFSRHG (SEQ ID NO. 56) | IWYDGRNK (SEQ ID NO. 57) | AREGITMVRGVIPL FDY (SEQ ID NO. 58) |
| TE03 | GFTFSRYG (SEQ ID NO. 59) | IWYDGSYK (SEQ ID NO. 60) | ARFGILTGYYFDY (SEQ ID NO. 61) |
| TE04 | GFTFSRHG (SEQ ID NO. 56) | IWYDGRNK (SEQ ID NO. 57) | AREGITMVRGVIPL FDY |
| | | | (SEQ ID NO. 58) |
| TE07 | GFTFSSYA (SEQ ID NO. 62) | ISGSGYST (SEQ ID NO. 63) | AKGKVGPTYAFDL (SEQ ID NO. 64) |
| TE10 | GFTFSSYG (SEQ ID NO. 65) | IWYDGSNK (SEQ ID NO. 66) | AREGRGMDV (SEQ ID NO. 67) |
| TM1 | GFTFSTYG (SEQ ID NO. 68) | IWYDGYNK (SEQ ID NO. 69) | EGWFGKLLSALDI (SEQ ID NO. 70) |

| **Table 2: VL CDR Sequences** | | | |
|---|---|---|---|
| **Antibody** | **CDR1** | **CDR2** | **CDR3** |
| TA02 | QASQDISNYLN (SEQ ID NO. 14) | AASSLQS (SEQ ID NO. 15) | QQSYSTPT (SEQ ID NO. 16) |
| TC01 | SGDKLGNKNAY (SEQ ID NO. 17) | QSTRRPS (SEQ ID NO. 18) | QAWDSSSGWEV (SEQ ID NO. 19) |
| TC02 | GASQSVSSSYLA (SEQ ID NO. 20) | DASSRAT (SEQ ID NO. 21) | HQYNNWPRT (SEQ ID NO. 22) |
| TD01 | SGSSSNIGSNYVY (SEQ ID NO. 23) | RNNQRPS (SEQ ID NO. 24) | AAWDDSLNGVV (SEQ ID NO. 25) |
| TE01 | KSSQSVLDSSNNKNYVA (SEQ ID NO. 26) | WASTRES (SEQ ID NO. 27) | QQYYTTRWT (SEQ ID NO. 28) |
| TG02 | RASQGIRNDLG (SEQ ID NO. 29) | DASNLET (SEQ ID NO. 30) | QQYDNLPLT (SEQ ID NO. 31) |
| TC03 | SGDKLGNKNAY (SEQ ID NO. 17) | QSTRRPS (SEQ ID NO. 18) | QAWLSSSGWEV (SEQ ID NO. 166) |
| TC04 | SGDKLGNKNAY (SEQ ID NO. 17) | QSTRRPS (SEQ ID NO. 18) | QAWDSSSGWEV (SEQ ID NO. 167) |
| TC05 | SGDKLGNKNAY (SEQ ID NO. 17) | QSTRRPS (SEQ ID NO. 18) | QAWDSSSGWEV (SEQ ID NO. 19) |
| TE02.1 | SGHSSYA (SEQ ID NO. 71) | LNSDGSH (SEQ ID NO. 72) | QTWGTGMLC (SEQ ID NO. 73) |
| TE02.2 | SGHSSYA (SEQ ID NO. 71) | LNSDGSH (SEQ ID NO. 72) | QTWGTGMLF (SEQ ID NO. 74) |
| TE02.3 | SGHSSYA (SEQ ID NO. 71) | LNSDGSH (SEQ ID NO. 72) | QTWGTGMLW (SEQ ID NO. 75) |
| TE03 | SGHSSYA | LNSDGSH | QTWGTGCC |
| | (SEQ ID NO. 71) | (SEQ ID NO. 72) | (SEQ ID NO. 76) |
| TE04 | SGSVSTSYN (SEQ ID NO. 77) | STN (SEQ ID NO. 78) | VLYMGSGII (SEQ ID NO. 79) |
| TE07 | SGSVSTSYY (SEQ ID NO. 80) | STN (SEQ ID NO. 78) | VLYMGSGTCC (SEQ ID NO. 81) |
| TE10 | SGHSSYI (SEQ ID NO. 82) | LEGSGSY (SEQ ID NO. 83) | ETWDSNTPHAV (SEQ ID NO. 84) |
| TM1 | QGIRND (SEQ ID NO. 85) | PAS (SEQ ID NO. 86) | LQDYNYPFT (SEQ ID NO. 87) |

CDR definition is also provided using annotation tool from http://www.abysis.org/ based on full VH and VL amino acid sequences as defined in Tables 7 and 8.

Below is shown the example in reference to SEQ ID No. 33 (VH of TC01).

**Table 3: Kabat defined CDR sequences**

| Region | Sequence Fragment | Residues |
|---|---|---|
| HFR1 | QIQLVQSGAEVKKPGASVKVSCKASGYTFT (SEQ ID No. 127) | 1 - 30 |
| CDR-H1 | SYGIS (SEQ ID No. 8) | 31 - 35 |
| HFR2 | VWRQAPGQGLEWMG (SEQ ID No. 128) | 36 - 49 |
| CDR-H2 | WISAYNGNTNYAQKLQG (SEQ ID No. 5) | 50 - 66 |
| HFR3 | RVTMTTDTSTSTAYMELRSLRSDDTAVYYCAR (SEQ ID No. 129) | 67 - 98 |
| CDR-H3 | WGRWLAHDY (SEQ ID No. 6) | 99 - 107 |
| HFR4 | WGQGTLVTVSS (SEQ ID No. 130) | 108 - 118 |

The VH amino acid sequence of any antibody may also be plugged into the annotation tool and IMGT defined CDR sequences are provided.

Below is shown the example in reference to SEQ ID No. 33 (VH of TC01).

**Table 4: IMGT defined CDR sequences**

| Region | Sequence Fragment | Residues |
|---|---|---|
| HFR1 | QIQLVQSGAEVKKPGASVKVSCKAS (SEQ ID No. 131) | 1 - 25 |
| CDR-H1 | GYTFTSYG (SEQ ID No. 132) | 26 - 33 |
| HFR2 | ISWVRQAPGQGLEWMGW (SEQ ID No. 133) | 34 - 50 |
| CDR-H2 | ISAYNGNT (SEQ ID No. 134) | 51 - 58 |
| HFR3 | NYAQKLQGRVTMTTDTSTSTAYMELRSLRSDDTAVYYC (SEQ ID No. 135) | 59 - 96 |
| CDR-H3 | ARWGRWLAHDY (SEQ ID No. 136) | 97 - 107 |
| HFR4 | WGQGTLVTVSS (SEQ ID No. 137) | 108 - 118 |

In addition, the VH amino acid sequence of any antibody disclosed herein may also be plugged into the annotation tool and the "All, side by side" defined CDR sequences are provided.

Below is shown the example in reference to SEQ ID No. 33 (VH of TC01).

**Table 5: All, side by side defined CDR sequences**

| Region | Definition | Sequence Fragment | Residues |
|---|---|---|---|
| HFR1 | Chothia | QIQLVQSGAEVKKPGASVKVSCKAS-----(SEQ ID No. 131) | 1 - 25 |
| | AbM | QIQLVQSGAEVKKPGASVKVSCKAS-----(SEQ ID No. 131) | 1 - 25 |
| | Kabat | QIQLVQSGAEVKKPGASVKVSCKASGYTFT (SEQ ID No. 127) | 1 - 30 |
| | Contact | QIQLVQSGAEVKKPGASVKVSCKASGYTF-(SEQ ID No. 138) | 1 - 29 |
| | IMGT | QIQLVQSGAEVKKPGASVKVSCKAS-----(SEQ ID No. 131) | 1 - 25 |
| CDR-H1 | Chothia | GYTFTSY---(SEQ ID No. 139) | 26 - 32 |
| | AbM | GYTFTSYGIS (SEQ ID No. 140) | 26 - 35 |
| | Kabat | -----SYGIS (SEQ ID No. 8) | 31 - 35 |
| | Contact | ----TSYGIS (SEQ ID No. 141) | 30 - 35 |
| | IMGT | GYTFTSYG--(SEQ ID No. 132) | 26 - 33 |
| HFR2 | Chothia | GISVWRQAPGQGLEWMGWI (SEQ ID No. 142) | 33 - 51 |
| | AbM | ---WVRQAPGQGLEWMG-- (SEQ ID No. 128) | 36 - 49 |
| | Kabat | ---WVRQAPGQGLEWMG--(SEQ ID No. 128) | 36 - 49 |
| | Contact | ---WVRQAPGQGLE-----(SEQ ID No. 143) | 36 - 46 |
| | IMGT | -ISWVRQAPGQGLEWMGW-(SEQ ID No. 133) | 34 - 50 |
| CDR-H2 | Chothia | -----SAYNGN (SEQ ID No. 144) | 52 - 57 |
| | AbM | ---WISAYNGNTN-------(SEQ ID No. 145) | 50 - 59 |
| | Kabat | ---WISAYNGNTNYAQKLQG (SEQ ID No. 5) | 50 - 66 |
| | Contact | WMGWISAYNGNTN------- (SEQ ID No. 146) | 47 - 59 |
| | IMGT | ----ISAYNGNT (SEQ ID No. 134) | 51 - 58 |
| HFR3 | Chothia | TNYAQKLQGRVTMTTDTSTSTAYMELRSLRSDDTAVYYCAR (SEQ ID No. 147) | 58 - 98 |
| | AbM | --YAQKLQGRVTMTTDTSTSTAYMELRSLRSDDTAVYYCAR (SEQ ID No. 148) | 60 - 98 |
| | Kabat | RVTMTTDTSTSTAYMELRSLRSDDTAVYYCAR (SEQ ID No. 129) | 67 - 98 |
| | Contact | --YAQKLQGRVTMTTDTSTSTAYMELRSLRSDDTAVYYC-(SEQ ID No. 149) | 60 - 96 |
| | IMGT | -NYAQKLQGRVTMTTDTSTSTAYMELRSLRSDDTAVYYC-(SEQ ID No. 135) | 59 - 96 |
| CDR-H3 | Chothia | --WGRWLAHDY (SEQ ID No. 6) | 99 - 107 |
| | AbM | --WGRWLAHDY (SEQ ID No. 6) | 99 - 107 |
| | Kabat | --WGRWLAHDY (SEQ ID No. 6) | 99 - 107 |
| | Contact | ARWGRWLAHD-(SEQ ID No. 150) | 97 - 106 |
| | IMGT | ARWGRWLAHDY (SEQ ID No. 136) | 97 - 107 |
| HFR4 | Chothia | -WGQGTLVTVSS (SEQ ID No. 137) | 108 - 118 |
| | AbM | -WGQGTLVTVSS (SEQ ID No. 137) | 108 - 118 |
| | Kabat | -WGQGTLVTVSS (SEQ ID No. 137) | 108 - 118 |
| | Contact | YWGQGTLVTVSS (SEQ ID No. 151) | 107 - 118 |
| | IMGT | -WGQGTLVTVSS (SEQ ID No. 137) | 108 - 118 |

CDR definition provided using annotation tool from http://www.abysis.org/ based on full VL amino acid sequence of TC01 (SEQ ID No. 39) is also reported.

**Table 6: All, side by side defined CDR sequences**

| Region | Definition | Sequence Fragment | Residues |
|---|---|---|---|
| LFR1 | Chothia | QAVLTQPPSVSVSPGQTASITC------(SEQ ID No. 152) | 1 - 22 |
| | AbM | QAVLTQPPSVSVSPGQTASITC------(SEQ ID No. 152) | 1 - 22 |
| | Kabat | QAVLTQPPSVSVSPGQTASITC------(SEQ ID No. 152) | 1 - 22 |
| | Contact | QAVLTQPPSVSVSPGQTASITCSGDKLG (SEQ ID No. 153) | 1 - 28 |
| | IMGT | QAVLTQPPSVSVSPGQTASITCSGD---(SEQ ID No. 154) | 1 - 25 |
| CDR-L1 | Chothia | SGDKLGNKNAY--(SEQ ID No. 17) | 23 - 33 |
| | AbM | SGDKLGNKNAY--(SEQ ID No. 17) | 23 - 33 |
| | Kabat | SGDKLGNKNAY--(SEQ ID No. 17) | 23 - 33 |
| | Contact | ------NKNA YWY (SEQ ID No. 155) | 29 - 35 |
| | IMGT | ---KLGNKN----(SEQ ID No. 156) | 26 - 31 |
| LFR2 | Chothia | --WYQQKPGQSPVLVMY (SEQ ID No. 156) | 34 - 48 |
| | AbM | --WYQQKPGQSPVLVMY (SEQ ID No. 156) | 34 - 48 |
| | Kabat | --WYQQKPGQSPVLVMY (SEQ ID No. 156) | 34 - 48 |
| | Contact | ----QQKPGQSPV----(SEQ ID No. 157) | 36 - 44 |
| | IMGT | AYWYQQKPGQSPVLVMY (SEQ ID No. 158) | 32 - 48 |
| CDR-L2 | Chothia | ---- QSTRRPS (SEQ ID No. 18) | 49 - 55 |
| | AbM | ----QSTRRPS (SEQ ID No. 18) | 49 - 55 |
| | Kabat | ---- QSTRRPS (SEQ ID No. 18) | 49 - 55 |
| | Contact | LVMYQSTRRP-(SEQ ID No. 159) | 45 - 54 |
| | IMGT | ----QS----- | 49 - 50 |
| LFR3 | Chothia | ----- GIPERFSASNSGNTATLTISGTQAMDEADYYC (SEQ ID No. 160) | 56 - 87 |
| | AbM | ----- GIPERFSASNSGNTATLTISGTQAMDEADYYC (SEQ ID No. 160) | 56 - 87 |
| | Kabat | ----- GIPERFSASNSGNTATLTISGTQAMDEADYYC(SEQ ID No. 160) | 56 - 87 |
| | Contact | ----SGIPERFSASNSGNTATLTISGTQAMDEADYYC (SEQ ID No. 161) | 55 - 87 |
| | IMGT | TRRPSGIPERFSASNSGNTATLTISGTQAMDEADYYC (SEQ ID No. 162) | 51 - 87 |
| CDR-L3 | Chothia | QAWDSSSGWEV (SEQ ID No. 19) | 88 - 98 |
| | AbM | QAWDSSSGWEV (SEQ ID No. 19) | 88 - 98 |
| | Kabat | QAWDSSSGWEV (SEQ ID No. 19) | 88 - 98 |
| | Contact | QAWDSSSGWE- (SEQ ID No. 163) | 88 - 97 |
| | IMGT | QAWDSSSGWEV (SEQ ID No. 19) | 88 - 98 |
| LFR4 | Chothia | -FGGGTKLTVL (SEQ ID No. 164) | 99 - 108 |
| | AbM | -FGGGTKLTVL (SEQ ID No. 164) | 99 - 108 |
| | Kabat | -FGGGTKLTVL (SEQ ID No. 164) | 99 - 108 |
| | Contact | VFGGGTKLTVL (SEQ ID No. 165) | 98 - 108 |
| | IMGT | -FGGGTKLTVL (SEQ ID No. 164) | 99 - 108 |

| **Table 7: VH amino acid sequences of exemplified antibodies** | |
|---|---|
| **Antibody** | **AA of VH** |
| TA02 | QVQLVQSGAEVKRPGSSVKVSCKASGGTFSSYAISWVRQAPGQGLEWMG GIIPIFGTANYAQKFQGRVTITADESTSTAYMELSNLRSEDTAVYYCARGDIA AAGRKGLPIYYMDVWGKGTTVTVSS (SEQ ID NO. 32) |
| TC01 | QIQLVQSGAEVKKPGASVKVSCKASGYTFTSYGISWVRQAPGQGLEWMG WISAYNGNTNYAQKLQGRVTMTTDTSTSTAYMELRSLRSDDTAVYYCARW GRWLAHDYWGQGTLVTVSS (SEQ ID NO. 33) |
| TC02 | QMQLVQSGAEVKKPGSSVKVSCKASGGTFSSYAISWVRQAPGQGLEWMG GIIPIFGTANYAQKFQGRVTITADESTSTAYMELSSLRSEDTAVYYCASPSGY YIYDAFDIWGQGTMVTVSS (SEQ ID NO. 34) |
| TD01 | QVQLVQSGAEVKKPGASVKVSCKASGYTFTSYGISWVRQAPGQGLEWMG WISAYNGNTNYAQKLQGRVTMTTDTSTSTAYMELRSLRSDDTAVYYCARDL GWPDDYWGQGTLVTVSS (SEQ ID NO. 35) |
| TE01 | EVQLLESGGGVVRPGGSLRLSCAASGFTFDDYGMSWVRQAPGKGLEWVS GINWNGGSTGYADSVKGRFTISRDNSKNTLYLQMNSLRAEDTAAYYCAKDR LRYCSSTSCYIPDYWGQGTLVTVSS (SEQ ID NO. 36) |
| TG02 | QVQLVQSGAEVKKPGASVKVSCKASGYTFTSYGISWVRQAPGQGLEWMG WISAYNGNTNYAQKLQGRVTMTTDTSTSTAYMELRSLRSDDTAVYYCARV GSTYDFWSGAYYYYGMDVWGQGTTVTVSS (SEQ ID NO. 37) |
| TC03 | QIQLVQSGAEVKQPGASVSVSCAASGYTFTSYGISWVRQAPGQGLEWMG WISAYNGNTNYAQKLQGRVTMTTDTSTSTAYMELSSLRSDDTAVYYCARW GRWLAHDYWGQGTLVTVSS (SEQ ID NO. 168) |
| TC04 | QIQLVQSGAEVKQPGASVSVSCAASGYTFTSYGISWVRQAPGQGLEWMG WISAYNGNTNYAQKLQGRVTMTTDTSTSTAYMELSSLRSDDTAVYYCARW GRWLAHDYWGQGTLVTVSS (SEQ ID NO. 169) |
| TC05 | QIQLVQSGAEVKQPGASVSVSCAASGYTFTSYGISWVRQAPGQGLEWMG WISAYNGNTNYAQKLQGRVTMTTDTSTSTAYMELSSLRSDDTAVYYCARW GRWLAHDYWGQGTLVTVSS (SEQ ID NO. 170) |
| TE02.1 | QVQLVESGGGVVQPGRSLRLSCAASGFTFSRHGMHWVRQAPGKGLEWVA VIWYDGRNKYYADSVKGRFTISRDNSKNTLYLQMNSLRVEDTAVYYCAREG ITMVRGVIPLFDYWGQGTLVTVSS (SEQ ID NO. 88) |
| TE02.2 | QVQLVESGGGVVQPGRSLRLSCAASGFTFSRHGMHWVRQAPGKGLEWVA VIWYDGRNKYYADSVKGRFTISRDNSKNTLYLQMNSLRVEDTAVYYCAREG ITMVRGVIPLFDYWGQGTLVTVSS (SEQ ID NO. 89) |
| TE02.3 | QVQLVESGGGVVQPGRSLRLSCAASGFTFSRHGMHWVRQAPGKGLEWVA VIWYDGRNKYYADSVKGRFTISRDNSKNTLYLQMNSLRVEDTAVYYCAREG ITMVRGVIPLFDYWGQGTLVTVSS (SEQ ID NO. 90) |
| TE03 | QVQLVESGGGVVQPGRSLRLSCAASGFTFSRYGMHWVRQAPGKGLEWVA VIWYDGSYKYYADSIKGRFTVSRDNSKNTLYLQMNSLRAEDTALYYCARFGI LTGYYFDYWGQGTLVTVSS (SEQ ID NO. 91) |
| TE04 | QVQLVESGGGVVQPGRSLRLSCAASGFTFSRHGMHWVRQAPGKGLEWVA VIWYDGRNKYYADSVKGRFTISRDNSKNTLYLQMNSLRVEDTAVYYCAREG ITMVRGVIPLFDYWGQGTLVTVSS (SEQ ID NO. 92) |
| TE07 | EVQLLESGGGLVQPGGSLRLSCAASGFTFSSYAMSWVRQAPGEGLEWVS GISGSGYSTYSADSVKGRFTIFKDNSKNTLYLQINSLRAEDTAVYYCAKGKV GPTYAFDLWGQGTMVTVSS (SEQ ID NO. 93) |
| TE10 | QVQLVESGGGVVQPGRSLRLSCAASGFTFSSYGMHWVRQAPGKGLEWVA VIWYDGSNKYYADSVKGRFTISRDNSKNTLYLQMNSLRAEDTAVYYCAREG RGMDVWGQGTTVTVSS (SEQ ID NO. 94) |
| TM1 | QVQLVESGGGVVQPGRSLRLSCAASGFTFSTYGIHWVRQAPGKGLEWVAV IWYDGYNKYYVDSVKGRFTISRDNSENTVYLQMNSLRTEDTAVYYCAREG WFGKLLSALDIWGQGTMVTVSS (SEQ ID NO. 95) |

| **Table 8: VL amino acid sequences of exemplified antibodies** | |
|---|---|
| **Antibody** | **AA of VL** |
| TA02 | DIQMTQSPSSLSASVGDRVTITCQASQDISNYLNWYQQKPGKAPKLLIY AASSLQSGVPSRFSGSGSGTDFTLTISSLQPEDFATYYCQQSYSTPTF GQGTKLEIK (SEQ ID NO. 38) |
| TC01 | QAVLTQPPSVSVSPGQTASITCSGDKLGNKNAYWYQQKPGQSPVLVM YQSTRRPSGIPERFSASNSGNTATLTISGTQAMDEADYYCQAWDSSS GWEVFGGGTKLTVL (SEQ ID NO. 39) |
| TC02 | ETTLTQSPATLSLSPGERATLSCGASQSVSSSYLAWYQQKPGLAPRLL IYDASSRATGIPDRFSGSGSGTDFTLTISRLEPEDFAVYYCHQYNNWP RTFGQGTKVEIK (SEQ ID NO. 40) |
| TD01 | QPVLTQPPSASGTPGQRVTISCSGSSSNIGSNYVYWYQQLPGTAPKLL IYRNNQRPSGVPDRFSGSKSGTSASLAISGLRSEDEADYYCAAWDDS LNGVVFGGGTKLTVL (SEQ ID NO. 41) |
| TE01 | DIQMTQSPDSLAVSLGERATINCKSSQSVLDSSNNKNYVAWFQQKPG QPPKLLIYWASTRESGVPDRFSGSGSGTDFTLTISSLQAEDVAVYYCQ QYYTTRWTFGQGTKVEIK (SEQ ID NO. 42) |
| TG02 | DIVMTQSPSSLSASVGDRVTITCRASQGIRNDLGWYQQKPGKAPKLLI YDASNLETGVPSRFSGSGSGTDFTFTISSLQPEDIATYYCQQYDNLPLT FGGGTKVEIK (SEQ ID NO. 43) |
| TC03 | QAVLTQPPSVSVSPGQTASITCSGDKLGNKNAYWYQQKPGQSPVLVM YQSTRRPSGIPERFSASNSGNTATLTISGTQAMDEADYYCQAWLSSS GWEVFGGGTKLTVL (SEQ ID NO. 171) |
| TC04 | QAVLTQPPSVSVSPGQTASITCSGDKLGNKNAYWYQQKPGQSPVLVM YQSTRRPSGIPERFSASNSGNTATLTISGTQAEDEADYYCQAWDSSS GWEVFGGGTKLTVL (SEQ ID NO. 172) |
| TC05 | QAVLTQPPSVSVSPGQTASITCSGDKLGNKNAYWYQQKPGQSPVLVM YQSTRRPSGIPERFSASNSGNTATLTISGTQAMDEADYYCQAWDSSS GWEVFGGGTKLTVL (SEQ ID NO. 173) |
| TE02.1 | QFVLTQSPSASASLGASVKLTCTLSSGHSSYAIAWHHQQPEKGPRYL MKLNSDGSHSKGDGIPDRFSGSSSGAERYLTISSLQSEDEADYYCQT WGTGMLCFGGGTQLTALR (SEQ ID NO. 96) |
| TE02.2 | QFVLTQSPSASASLGASVKLTCTLSSGHSSYAIAWHHQQPEKGPRYL MKLNSDGSHSKGDGIPDRFSGSSSGAERYLTISSLQSEDEADYYCQT WGTGMLFGGGTQLTALR (SEQ ID NO. 97) |
| TE02.3 | QFVLTQSPSASASLGASVKLTCTLSSGHSSYAIAWHHQQPEKGPRYL MKLNSDGSHSKGDGIPDRFSGSSSGAERYLTISSLQSEDEADYYCQT WGTGMLWFGGGTQLTALR (SEQ ID NO. 98) |
| TE03 | QVVLTQSPPASASLGASVKLTCTLSSGHSSYAIAWHQQQPEKGPRYL MKLNSDGSHSKGDGIPDRFSGSSSGAERYLTISSLQSEDEADYYCQT WGTGCCFGGGTQLTALR (SEQ ID NO. 99) |
| TE04 | QTVVTQESSFSVSPGGTVTLTCGLSSGSVSTSYNPSWYQQTPGQAPR TLIYSTNTRSSGVPDRFSGSILGNKAALTITGAQADDESDYYCVLYMGS GIIFGSGTKVTVL (SEQ ID NO. 100) |
| TE07 | QTVVTQEPSFSVSPGGTVTLTCGLSSGSVSTSYYPSWYQQTPGQAPR TLIYSTNTRSSGVPERFSGSILGNKAALTITGAQADDESDYYCVLYMGS GTCCFGGGTQLTALR (SEQ ID NO. 101) |
| TE10 | QPVLTQSSSASASLGSSVKLTCTLSSGHSSYIIAWHQQQPGKAPRYLM KLEGSGSYNKGSGVPDRFSGSSSGADRYLTISNLQFEDEADYYCETW DSNTPHAVFGGGTQLTAL (SEQ ID NO. 102) |
| TM1 | VIQMTQSPSSLSASVGDRVTITCRASQGIRNDLGWYLQKPGKAPELLIY PASSLQSGVPSRFSGSGSGTDFTLTISSLQPEDFATYYCLQDYNYPFT FGQGTKLEIK (SEQ ID NO. 103) |

| **Table 9: VH nucleotide sequences of exemplified antibodies** | |
|---|---|
| **Antibody** | **DNA of VH** |
| TA02 | CAGGTCCAGCTGGTACAGTCTGGGGCTGAGGTGAAGAGGCCTGG GTCCTCGGTGAAGGTCTCCTGCAAGGCTTCTGGAGGCACCTTCAG CAGCTATGCTATCAGCTGGGTGCGACAGGCCCCTGGACAAGGGCT TGAGTGGATGGGAGGGATCATCCCTATCTTTGGTACAGCAAACTAC GCACAGAAGTTCCAGGGCAGAGTCACGATTACCGCGGACGAATCC ACGAGCACAGCCTACATGGAGCTGAGCAACCTGAGATCTGAGGAC ACGGCCGTGTATTACTGTGCGAGAGGGGATATAGCAGCAGCTGGT AGGAAAGGACTGCCCATCTACTACATGGACGTCTGGGGCAAAGGG ACCACGGTCACCGTCTCCTCA (SEQ ID NO. 44) |
| TC01 | CAAATCCAGCTGGTACAGTCTGGGGCTGAGGTGAAGAAGCCTGGG GCCTCAGTGAAGGTCTCCTGCAAGGCTTCTGGTTACACCTTTACCA GCTACGGTATCAGCTGGGTGCGACAGGCCCCTGGACAAGGGCTTG AGTGGATGGGATGGATCAGCGCTTACAATGGTAACACAAACTATGC ACAGAAGCTCCAGGGCAGAGTCACCATGACCACAGACACATCCAC GAGCACAGCCTACATGGAGCTGAGGAGCCTGAGATCTGACGACAC GGCCGTGTATTACTGTGCGAGGTGGGGTAGGTGGCTGGCTCATGA CTACTGGGGCCAGGGAACCCTGGTCACCGTCTCCTCA (SEQ ID NO. 45) |
| TC02 | CAGATGCAGCTGGTGCAGTCTGGGGCTGAGGTGAAGAAGCCTGG GTCCTCGGTGAAGGTCTCCTGCAAGGCTTCTGGAGGCACCTTCAG CAGCTATGCTATCAGCTGGGTGCGACAGGCCCCTGGACAAGGGCT TGAGTGGATGGGAGGGATCATCCCTATCTTTGGTACAGCAAACTAC GCACAGAAGTTCCAGGGCAGAGTCACGATTACCGCGGACGAATCC ACGAGCACAGCCTACATGGAGCTGAGCAGCCTGAGATCTGAGGAC ACGGCCGTGTATTACTGTGCGAGCCCAAGTGGTTATTATATTTATG ATGCTTTTGATATCTGGGGCCAAGGGACAATGGTCACCGTCTCTTC A (SEQ ID NO. 46) |
| TD01 | CAGGTCCAGCTGGTGCAGTCTGGAGCTGAGGTGAAGAAGCCTGGG GCCTCAGTGAAGGTCTCCTGCAAGGCTTCTGGTTACACCTTTACCA GCTATGGTATCAGCTGGGTGCGACAGGCCCCTGGACAAGGGCTTG AGTGGATGGGATGGATCAGCGCTTACAATGGTAACACAAACTATGC ACAGAAGCTCCAGGGCAGAGTCACCATGACCACAGACACATCCAC GAGCACAGCCTACATGGAGCTGAGGAGCCTGAGATCTGACGACAC GGCCGTGTATTACTGTGCGAGAGATCTCGGCTGGCCAGATGACTA CTGGGGCCAGGGAACCCTGGTCACCGTCTCCTCA (SEQ ID NO. 47) |
| TE01 | GAAGTGCAGTTGTTGGAGTCTGGGGGAGGTGTGGTACGGCCTGG GGGGTCCCTGAGACTCTCCTGTGCAGCCTCTGGATTCACCTTTGAT GATTATGGCATGAGCTGGGTCCGCCAAGCTCCAGGGAAGGGGCTG GAGTGGGTCTCTGGTATTAATTGGAATGGTGGTAGCACAGGTTATG CAGACTCCGTGAAGGGCCGATTCACCATCTCCAGAGACAATTCCAA GAACACGCTGTATCTGCAAATGAACAGCCTGAGAGCTGAGGACAC GGCTGCGTATTACTGTGCGAAAGATCGACTAAGATATTGTAGTAGT |
| | ACCAGCTGCTATATCCCTGACTACTGGGGCCAGGGAACCCTGGTC ACCGTCTCCTCA (SEQ ID NO. 48) |
| TG02 | CAGGTGCAGCTGGTGCAGTCTGGAGCTGAGGTGAAGAAGCCTGG GGCCTCAGTGAAGGTCTCCTGCAAGGCTTCTGGTTACACCTTTACC AGCTATGGTATCAGCTGGGTGCGACAGGCCCCTGGACAAGGGCTT GAGTGGATGGGATGGATCAGCGCTTACAATGGTAACACAAACTATG CACAGAAGCTCCAGGGCAGAGTCACCATGACCACAGACACATCCA CGAGCACAGCCTACATGGAGCTGAGGAGCCTGAGATCTGACGACA CGGCCGTGTATTACTGTGCGAGAGTAGGGAGCACTTACGATTTTTG GAGTGGCGCCTACTACTACTACGGTATGGACGTCTGGGGCCAAGG GACCACGGTCACCGTCTCCTCA (SEQ ID NO. 49) |
| TC03 | CAGATCCAGTTGGTGCAAAGTGGGGCTGAGGTAAAGCAGCCGGGT GCAAGTGTGTCCGTAAGTTGTGCAGCCAGTGGCTACACCTTTACTA GTTACGGAATTTCATGGGTGCGGCAAGCTCCCGGTCAGGGATTGG AATGGATGGGATGGATTTCAGCATACAACGGGAACACAAATTACGC TCAAAAATTGCAGGGTCGAGTTACCATGACTACAGACACGTCTACG TCTACAGCTTACATGGAACTTTCCAGCCTGCGGTCCGACGACACCG CAGTTTATTATTGCGCCCGCTGGGGGAGATGGCTCGCGCATGACT ACTGGGGGCAGGGCACGCTGGTTACCGTTTCCTCAGCCTCCACCA AGGGCCCATCCGTCTTCCCCCTGGCGCCCTGCTCCAGGAGCACCT CCGAGAGCACAGCCGCCCTGGGCTGCCTGGTCAAGGACTACTTCC CCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTGGTGACCGTGCCCTCCAGCAGCTTGGGCACGA AGACCTACACCTGCAACGTAGATCACAAGCCCAGCAACACCAAGGT GGACAAGAGAGTTGAGTCCAAATATGGTCCCCCATGCCCACCCTG CCCAGCACCTGAGTTCGAGGGGGGACCATCAGTCTTCCTGTTCCC CCCAAAACCCAAGGACACTCTCATGATCTCCCGGACCCCTGAGGT CACGTGCGTGGTGGTGGACGTGAGCCAGGAAGACCCCGAGGTCC AGTTCAACTGGTACGTGGATGGCGTGGAGGTGCATAATGCCAAGA CAAAGCCGCGGGAGGAGCAGTTCAACAGCACGTACCGTGTGGTCA GCGTCCTCACCGTCCTGCACCAGGACTGGCTGAACGGCAAGGAGT ACAAGTGCAAGGTGTCCAACAAAGGCCTCCCGTCCTCCATCGAGA AAACCATCTCCAAAGCCAAAGGGCAGCCCCGAGAGCCACAGGTGT |
| | ACACCCTGCCCCCATCCCAGGAGGAGATGACCAAGAACCAGGTCA GCCTGACCTGCCTGGTCAAAGGCTTCTACCCCAGCGACATCGCCG TGGAGTGGGAGAGCAATGGGCAGCCGGAGAACAACTACAAGACCA CGCCTCCCGTGCTGGACTCCGACGGCTCCTTCTTCCTCTACAGCA GGCTAACCGTGGACAAGAGCAGGTGGCAGGAGGGGAATGTCTTCT CATGCTCCGTGATGCATGAGGCTCTGCACAACCACTACACACAGAA GAGTCTCTCCCTGTCTCTGGGTAAA (SEQ ID NO. 174) |
| TC04 | CAGATCCAGTTGGTGCAAAGTGGGGCTGAGGTAAAGCAGCCGGGT GCAAGTGTGTCCGTAAGTTGTGCAGCCAGTGGCTACACCTTTACTA GTTACGGAATTTCATGGGTGCGGCAAGCTCCCGGTCAGGGATTGG AATGGATGGGATGGATTTCAGCATACAACGGGAACACAAATTACGC TCAAAAATTGCAGGGTCGAGTTACCATGACTACAGACACGTCTACG TCTACAGCTTACATGGAACTTTCCAGCCTGCGGTCCGACGACACCG CAGTTTATTATTGCGCCCGCTGGGGGAGATGGCTCGCGCATGACT ACTGGGGGCAGGGCACGCTGGTTACCGTTTCCTCAGCCTCCACCA AGGGCCCATCCGTCTTCCCCCTGGCGCCCTGCTCCAGGAGCACCT CCGAGAGCACAGCCGCCCTGGGCTGCCTGGTCAAGGACTACTTCC CCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTGGTGACCGTGCCCTCCAGCAGCTTGGGCACGA AGACCTACACCTGCAACGTAGATCACAAGCCCAGCAACACCAAGGT GGACAAGAGAGTTGAGTCCAAATATGGTCCCCCATGCCCACCCTG CCCAGCACCTGAGTTCGAGGGGGGACCATCAGTCTTCCTGTTCCC CCCAAAACCCAAGGACACTCTCATGATCTCCCGGACCCCTGAGGT CACGTGCGTGGTGGTGGACGTGAGCCAGGAAGACCCCGAGGTCC AGTTCAACTGGTACGTGGATGGCGTGGAGGTGCATAATGCCAAGA CAAAGCCGCGGGAGGAGCAGTTCAACAGCACGTACCGTGTGGTCA GCGTCCTCACCGTCCTGCACCAGGACTGGCTGAACGGCAAGGAGT ACAAGTGCAAGGTGTCCAACAAAGGCCTCCCGTCCTCCATCGAGA AAACCATCTCCAAAGCCAAAGGGCAGCCCCGAGAGCCACAGGTGT ACACCCTGCCCCCATCCCAGGAGGAGATGACCAAGAACCAGGTCA GCCTGACCTGCCTGGTCAAAGGCTTCTACCCCAGCGACATCGCCG TGGAGTGGGAGAGCAATGGGCAGCCGGAGAACAACTACAAGACCA CGCCTCCCGTGCTGGACTCCGACGGCTCCTTCTTCCTCTACAGCA |
| | GGCTAACCGTGGACAAGAGCAGGTGGCAGGAGGGGAATGTCTTCT CATGCTCCGTGATGCATGAGGCTCTGCACAACCACTACACACAGAA GAGTCTCTCCCTGTCTCTGGGTAAA (SEQ ID NO. 175) |
| TC05 | CAGATCCAGTTGGTGCAAAGTGGGGCTGAGGTAAAGCAGCCGGGT GCAAGTGTGTCCGTAAGTTGTGCAGCCAGTGGCTACACCTTTACTA GTTACGGAATTTCATGGGTGCGGCAAGCTCCCGGTCAGGGATTGG AATGGATGGGATGGATTTCAGCATACAACGGGAACACAAATTACGC TCAAAAATTGCAGGGTCGAGTTACCATGACTACAGACACGTCTACG TCTACAGCTTACATGGAACTTTCCAGCCTGCGGTCCGACGACACCG CAGTTTATTATTGCGCCCGCTGGGGGAGATGGCTCGCGCATGACT ACTGGGGGCAGGGCACGCTGGTTACCGTTTCCTCAGCCTCCACCA AGGGCCCATCCGTCTTCCCCCTGGCGCCCTGCTCCAGGAGCACCT CCGAGAGCACAGCCGCCCTGGGCTGCCTGGTCAAGGACTACTTCC CCGAACCGGTGACGGTGTCGTGGAACTCAGGCGCCCTGACCAGC GGCGTGCACACCTTCCCGGCTGTCCTACAGTCCTCAGGACTCTACT CCCTCAGCAGCGTGGTGACCGTGCCCTCCAGCAGCTTGGGCACGA AGACCTACACCTGCAACGTAGATCACAAGCCCAGCAACACCAAGGT GGACAAGAGAGTTGAGTCCAAATATGGTCCCCCATGCCCACCCTG CCCAGCACCTGAGTTCGAGGGGGGACCATCAGTCTTCCTGTTCCC CCCAAAACCCAAGGACACTCTCATGATCTCCCGGACCCCTGAGGT CACGTGCGTGGTGGTGGACGTGAGCCAGGAAGACCCCGAGGTCC AGTTCAACTGGTACGTGGATGGCGTGGAGGTGCATAATGCCAAGA CAAAGCCGCGGGAGGAGCAGTTCAACAGCACGTACCGTGTGGTCA GCGTCCTCACCGTCCTGCACCAGGACTGGCTGAACGGCAAGGAGT ACAAGTGCAAGGTGTCCAACAAAGGCCTCCCGTCCTCCATCGAGA AAACCATCTCCAAAGCCAAAGGGCAGCCCCGAGAGCCACAGGTGT ACACCCTGCCCCCATCCCAGGAGGAGATGACCAAGAACCAGGTCA GCCTGACCTGCCTGGTCAAAGGCTTCTACCCCAGCGACATCGCCG TGGAGTGGGAGAGCAATGGGCAGCCGGAGAACAACTACAAGACCA CGCCTCCCGTGCTGGACTCCGACGGCTCCTTCTTCCTCTACAGCA GGCTAACCGTGGACAAGAGCAGGTGGCAGGAGGGGAATGTCTTCT CATGCTCCGTGATGCATGAGGCTCTGCACAACCACTACACACAGAA GAGTCTCTCCCTGTCTCTGGGTAAA (SEQ ID NO. 176) |
| TE02.1 | CAGGTCCAACTCGTCGAGAGCGGAGGAGGAGTCGTCCAACCCGG AAGGAGCTTGCGGCTATCATGCGCGGCATCCGGCTTCACATTTTCC CGGCACGGGATGCACTGGGTCAGGCAAGCACCCGGCAAGGGGCT AGAATGGGTCGCGGTCATCTGGTATGATGGAAGGAACAAATACTAT GCCGACTCAGTCAAGGGGCGATTTACAATTTCGCGAGACAACTCCA AGAATACGCTATACCTGCAAATGAACTCGCTGAGGGTCGAGGACAC GGCGGTTTATTACTGCGCGAGGGAGGGGATAACTATGGTCAGAGG AGTCATTCCGCTATTTGACTATTGGGGGCAGGGTACCTTAGTCACG GTCTCGAGC (SEQ ID NO. 104) |
| TE02.2 | CAGGTCCAACTCGTCGAGAGCGGAGGAGGAGTCGTCCAACCCGG AAGGAGCTTGCGGCTATCATGCGCGGCATCCGGCTTCACATTTTCC CGGCACGGGATGCACTGGGTCAGGCAAGCACCCGGCAAGGGGCT AGAATGGGTCGCGGTCATCTGGTATGATGGAAGGAACAAATACTAT GCCGACTCAGTCAAGGGGCGATTTACAATTTCGCGAGACAACTCCA AGAATACGCTATACCTGCAAATGAACTCGCTGAGGGTCGAGGACAC GGCGGTTTATTACTGCGCGAGGGAGGGGATAACTATGGTCAGAGG AGTCATTCCGCTATTTGACTATTGGGGGCAGGGTACCTTAGTCACG GTCTCGAGC (SEQ ID NO. 105) |
| TE02.3 | CAGGTCCAACTCGTCGAGAGCGGAGGAGGAGTCGTCCAACCCGG AAGGAGCTTGCGGCTATCATGCGCGGCATCCGGCTTCACATTTTCC CGGCACGGGATGCACTGGGTCAGGCAAGCACCCGGCAAGGGGCT AGAATGGGTCGCGGTCATCTGGTATGATGGAAGGAACAAATACTAT GCCGACTCAGTCAAGGGGCGATTTACAATTTCGCGAGACAACTCCA AGAATACGCTATACCTGCAAATGAACTCGCTGAGGGTCGAGGACAC GGCGGTTTATTACTGCGCGAGGGAGGGGATAACTATGGTCAGAGG AGTCATTCCGCTATTTGACTATTGGGGGCAGGGTACCTTAGTCACG GTCTCGAGC (SEQ ID NO. 106) |
| TE03 | CAGGTCCAACTCGTTGAATCCGGGGGGGGAGTCGTCCAACCGGG GAGAAGCCTGCGGCTAAGCTGCGCGGCTTCGGGATTCACATTCTC TCGATACGGGATGCACTGGGTCAGGCAAGCACCGGGGAAGGGCTT GGAATGGGTCGCCGTCATATGGTACGACGGATCATATAAATATTAT GCTGACTCTATAAAGGGGCGATTCACGGTTAGCCGAGACAACTCCA AAAACACGCTATACCTGCAAATGAACTCACTGCGAGCTGAAGATAC GGCGCTATATTATTGCGCCCGATTCGGAATCCTGACCGGATATTAT |
| | TTTGACTACTGGGGGCAGGGTACCCTAGTCACGGTCTCGAGC (SEQ ID NO. 107) |
| TE04 | CAGGTCCAACTCGTCGAGAGCGGAGGAGGAGTCGTCCAACCCGG AAGGAGCTTGCGGCTATCATGCGCGGCATCCGGCTTCACATTTTCC CGGCACGGGATGCACTGGGTCAGGCAAGCACCCGGCAAGGGGCT AGAATGGGTCGCGGTCATCTGGTATGATGGAAGGAACAAATACTAT GCCGACTCAGTCAAGGGGCGATTTACAATTTCGCGAGACAACTCCA AGAATACGCTATACCTGCAAATGAACTCGCTGAGGGTCGAGGACAC GGCGGTTTATTACTGCGCGAGGGAGGGGATAACTATGGTCAGAGG AGTCATTCCGCTATTTGACTATTGGGGGCAGGGTACCTTAGTCACG GTCTCGAGC (SEQ ID NO. 108) |
| TE07 | GAAGTCCAACTGCTGGAATCGGGCGGGGGGCTGGTCCAACCCGG AGGATCATTGAGGCTATCATGCGCCGCTTCCGGCTTTACATTTAGC TCATACGCAATGTCATGGGTCCGACAAGCTCCGGGGGAGGGACTG GAATGGGTCTCTGGGATTTCGGGCTCTGGATACTCCACATATAGCG CGGACTCAGTCAAGGGGAGATTCACGATTTTTAAGGATAACTCCAA GAATACATTATATCTGCAAATAAACTCGCTGAGGGCGGAGGATACC GCCGTTTATTACTGCGCCAAAGGGAAAGTCGGGCCAACTTACGCAT TCGACCTATGGGGGCAGGGTACCATGGTCACGGTCTCGAGC (SEQ ID NO. 109) |
| TE10 | CAAGTGCAACTCGTGGAATCGGGCGGAGGGGTGGTGCAACCGGG AAGATCACTGCGACTATCATGCGCCGCATCGGGATTTACATTTAGC AGCTACGGGATGCACTGGGTCCGCCAAGCACCCGGAAAAGGGCT GGAATGGGTCGCGGTGATTTGGTACGATGGCTCGAATAAATACTAT GCTGACTCGGTGAAGGGCCGATTCACAATCTCGCGGGACAACTCC AAAAACACACTATATCTGCAAATGAACTCACTGCGGGCGGAGGATA CCGCGGTATATTACTGCGCGAGGGAGGGGCGCGGAATGGATGTAT GGGGGCAGGGTACCACGGTGACCGTCTCGAGC (SEQ ID NO. 110) |
| TM1 | CAGGTGCAGCTGGTGGAGTCTGGGGGAGGCGTGGTCCAGCCTGG GAGGTCCCTGAGACTCTCCTGTGCAGCATCTGGATTCACCTTCAGT ACCTATGGCATACACTGGGTCCGCCAGGCTCCAGGCAAGGGGCTG GAGTGGGTGGCAGTTATATGGTATGATGGATATAATAAATACTATGT AGACTCCGTGAAGGGCCGATTCACCATCTCCAGAGACAATTCCGA GAACACGGTGTATCTGCAGATGAACAGCCTGAGAACCGAGGACAC |
| | GGCTGTTTATTACTGTGCGAGAGAAGGATGGTTCGGGAAATTATTA TCCGCTCTTGATATCTGGGGCCAAGGGACAATGGTCACCGTCTCTT CA (SEQ ID NO. 111) |

| **Table 10: VL nucleotide sequences of exemplified antibodies** | |
|---|---|
| **Antibody** | **DNA of VL** |
| **TA02** | GACATCCAGATGACCCAGTCTCCATCCTCCCTGTCTGCATCTGTAG GAGACAGAGTCACCATCACTTGCCAGGCGAGTCAGGACATTAGCA ACTACTTAAATTGGTATCAGCAGAAACCAGGGAAAGCCCCTAAGCT CCTGATCTATGCTGCATCCAGTTTGCAAAGTGGGGTCCCATCAAGG TTCAGTGGCAGTGGATCTGGGACAGATTTCACTCTCACCATCAGCA GTCTGCAACCTGAAGATTTTGCAACTTACTACTGTCAACAGAGTTAC AGTACCCCTACTTTTGGCCAGGGGACCAAGCTGGAGATCAAA (SEQ ID NO. 50) |
| TC01 | CAGGCAGTGCTGACTCAGCCACCCTCAGTGTCCGTGTCCCCAGGA CAGACAGCCAGCATCACCTGCTCTGGAGATAAATTGGGAAATAAAA ATGCTTATTGGTATCAGCAGAAGCCAGGCCAGTCCCCTGTACTGGT CATGTATCAAAGTACCAGACGGCCCTCAGGGATCCCTGAGCGATTC TCTGCCTCCAACTCTGGGAACACAGCCACTCTGACCATCAGCGGG ACCCAGGCTATGGATGAGGCTGACTATTACTGTCAGGCGTGGGAC AGCAGTAGTGGATGGGAGGTATTCGGCGGAGGGACCAAGCTGACC GTCCTA (SEQ ID NO. 51) |
| TC02 | GAAACGACACTCACGCAGTCTCCAGCCACCCTGTCTTTGTCTCCAG GGGAAAGAGCCACCCTCTCCTGCGGGGCCAGTCAGAGTGTTAGCA GCAGCTACTTAGCCTGGTACCAGCAGAAACCTGGCCTGGCGCCCA GGCTCCTCATCTATGATGCATCCAGCAGGGCCACTGGCATCCCAG ACAGGTTCAGTGGCAGTGGGTCTGGGACAGACTTCACTCTCACCAT CAGCAGACTGGAGCCTGAAGATTTTGCAGTTTATTACTGTCACCAG TATAATAACTGGCCTAGGACGTTCGGCCAAGGGACCAAGGTGGAA ATCAAA (SEQ ID NO. 52) |
| TD01 | CAGCCTGTGCTGACTCAGCCACCCTCAGCGTCTGGGACCCCCGGG CAGAGGGTCACCATCTCTTGTTCTGGAAGCAGCTCCAACATCGGAA GTAATTATGTATACTGGTACCAGCAGCTCCCAGGAACGGCCCCCAA ACTCCTCATCTATAGGAATAATCAGCGGCCCTCAGGGGTCCCTGAC |
| | CGATTCTCTGGCTCCAAGTCTGGCACCTCAGCCTCCCTGGCCATCA GTGGGCTCCGGTCCGAGGATGAGGCTGATTATTATTGTGCAGCAT GGGATGACAGCCTGAATGGTGTGGTATTCGGCGGAGGGACCAAGC TGACCGTCCTA (SEQ ID NO. 53) |
| TE01 | GACATCCAGATGACCCAGTCTCCGGACTCCCTGGCTGTGTCTCTG GGCGAGAGGGCCACCATCAACTGCAAGTCCAGCCAGAGTGTTTTG GACAGCTCCAACAATAAAAATTATGTCGCTTGGTTCCAGCAGAAAC CAGGACAGCCTCCTAAGCTGCTCATTTACTGGGCCTCTACCCGGG AATCCGGGGTCCCTGACCGATTCAGTGGCAGCGGTTCTGGGACAG ATTTCACTCTCACCATCAGCAGCCTGCAGGCTGAAGATGTGGCAGT TTATTACTGTCAGCAATATTATACTACTCGGTGGACGTTCGGCCAAG GGACCAAGGTGGAAATCAAA (SEQ ID NO. 54) |
| TG02 | GACATCGTGATGACCCAGTCTCCATCCTCCCTGTCTGCATCTGTAG GAGACAGAGTCACCATCACTTGCCGGGCAAGTCAGGGCATTAGAA ATGATTTAGGCTGGTATCAGCAAAAACCAGGGAAAGCCCCTAAACT CCTGATCTACGATGCATCCAATTTGGAAACAGGGGTCCCATCAAGG TTCAGTGGAAGTGGATCTGGGACAGATTTTACTTTCACCATCAGCA GCCTGCAGCCTGAAGATATTGCAACATATTACTGTCAACAGTATGAT AACCTCCCGCTCACTTTCGGCGGAGGGACCAAGGTGGAGATCAAA (SEQ ID NO. 55) |
| TC03 | CAGGCGGTGCTTACTCAGCCCCCAAGTGTGTCTGTTTCCCCCGGT CAGACTGCGTCTATAACCTGCTCCGGGGATAAACTCGGCAACAAGA ATGCGTACTGGTACCAACAGAAGCCGGGACAGAGCCCAGTCTTGG TCATGTACCAATCCACCCGGAGACCTAGCGGCATTCCAGAGCGCTT TAGTGCATCTAATTCTGGCAATACGGCGACGTTGACCATCAGTGGT ACACAAGCGATGGACGAGGCAGATTACTACTGTCAGGCATGGCTG TCATCATCCGGGTGGGAGGTGTTTGGCGGCGGAACAAAACTCACT GTCCTAGGTCAGCCCAAGGCTGCACCAAGTGTCACTCTGTTCCCG CCCTCCTCTGAGGAGCTTCAAGCCAACAAGGCCACACTGGTGTGT CTCATAAGTGACTTCTACCCGGGAGCCGTGACAGTGGCCTGGAAG GCAGATAGCAGCCCCGTCAAGGCGGGAGTGGAGACCACCACACC CTCCAAACAAAGCAACAACAAGTACGCGGCCAGCAGCTATCTGAG CCTGACGCCTGAGCAGTGGAAGTCCCACAGAAGCTACAGCTGCCA |
| | GGTCACGCATGAAGGGAGCACCGTGGAGAAGACAGTGGCCCCTAC AGAATGTTCA (SEQ ID NO. 177) |
| TC04 | CAGGCCGTCTTGACTCAACCACCCTCCGTTAGTGTCTCCCCCGGC CAGACGGCGAGTATCACCTGTAGTGGTGATAAGCTGGGCAATAAG AATGCTTACTGGTACCAGCAAAAACCCGGACAGAGCCCAGTGCTG GTGATGTATCAGTCTACAAGACGACCTAGCGGCATCCCAGAAAGGT TTTCTGCCAGCAATTCTGGCAATACGGCGACGCTGACTATTAGTGG CACACAAGCAGAGGATGAGGCGGACTATTACTGCCAAGCATGGGA CAGTAGTAGTGGTTGGGAAGTCTTCGGGGGCGGCACTAAGCTCAC CGTCCTAGGTCAGCCCAAGGCTGCACCAAGTGTCACTCTGTTCCC GCCCTCCTCTGAGGAGCTTCAAGCCAACAAGGCCACACTGGTGTG TCTCATAAGTGACTTCTACCCGGGAGCCGTGACAGTGGCCTGGAA GGCAGATAGCAGCCCCGTCAAGGCGGGAGTGGAGACCACCACAC CCTCCAAACAAAGCAACAACAAGTACGCGGCCAGCAGCTATCTGA GCCTGACGCCTGAGCAGTGGAAGTCCCACAGAAGCTACAGCTGCC AGGTCACGCATGAAGGGAGCACCGTGGAGAAGACAGTGGCCCCTA CAGAATGTTCA (SEQ ID NO. 178) |
| TC05 | CAAGCTGTATTGACACAACCTCCTAGTGTCAGTGTAAGCCCTGGTC AAACTGCCTCCATTACTTGCTCTGGCGACAAGCTCGGAAATAAGAA CGCGTACTGGTACCAACAGAAGCCCGGACAGTCACCTGTGCTTGT TATGTATCAAAGCACCAGGAGACCTTCAGGGATACCAGAAAGGTTT AGTGCGTCTAATTCCGGGAATACCGCGACACTGACGATAAGCGGC ACTCAGGCTATGGACGAAGCGGATTACTACTGTCAGGCATGGGATT CATCATCAGGTTGGGAAGTATTCGGGGGCGGTACAAAATTGACGG TCCTAGGTCAGCCCAAGGCTGCACCAAGTGTCACTCTGTTCCCGC CCTCCTCTGAGGAGCTTCAAGCCAACAAGGCCACACTGGTGTGTCT CATAAGTGACTTCTACCCGGGAGCCGTGACAGTGGCCTGGAAGGC AGATAGCAGCCCCGTCAAGGCGGGAGTGGAGACCACCACACCCTC CAAACAAAGCAACAACAAGTACGCGGCCAGCAGCTATCTGAGCCT GACGCCTGAGCAGTGGAAGTCCCACAGAAGCTACAGCTGCCAGGT CACGCATGAAGGGAGCACCGTGGAGAAGACAGTGGCCCCTACAGA ATGTTCA (SEQ ID NO. 179) |
| TE02.1 | GCGGCCGCCATGAATTTTGGACTGAGGCTGATTTTCCTGGTGCTGA CCCTGAAAGGCGTCCAGTGTCAATTTGTGCTGACGCAATCGCCATC |
| | GGCTAGTGCATCACTGGGGGCGAGCGTTAAATTGACATGCACGCT ATCGAGCGGACACTCATCATATGCCATTGCATGGCACCACCAACAA CCGGAAAAGGGGCCACGATATCTAATGAAGCTGAACTCTGACGGA TCGCATTCGAAAGGGGATGGGATTCCCGACCGATTCTCGGGAAGC AGCAGCGGAGCTGAAAGATATTTAACGATATCATCGCTGCAATCGG AGGATGAAGCTGACTACTACTGCCAAACTTGGGGAACGGGGATGC TATGCTTCGGAGGAGGCACACAATTGACGGCCCTTGGACAACCG (SEQ ID NO. 112) |
| TE02.2 | GCGGCCGCCATGAATTTTGGACTGAGGCTGATTTTCCTGGTGCTGA CCCTGAAAGGCGTCCAGTGTCAATTTGTACTGACGCAAAGCCCAAG CGCGAGCGCATCGCTGGGAGCATCCGTCAAGCTCACATGCACGCT ATCATCGGGCCATTCAAGCTATGCCATAGCATGGCATCACCAACAA CCGGAGAAGGGACCTCGATATCTGATGAAGCTGAATAGCGACGGC TCCCACTCAAAGGGGGACGGAATCCCGGATAGATTTTCGGGCTCA TCAAGCGGAGCGGAGCGATATCTCACGATCTCTAGCCTGCAAAGC GAGGATGAGGCCGACTACTACTGCCAAACATGGGGGACGGGAATG CTATTCGGAGGAGGCACGCAACTGACGGCGCTGGGGCAACCA (SEQ ID NO. 113) |
| TE02.3 | GCGGCCGCCATGAATTTTGGACTGAGGCTGATTTTCCTGGTGCTGA CCCTGAAAGGCGTCCAGTGTCAATTTGTGCTGACGCAATCACCATC GGCTTCGGCGAGCCTGGGGGCATCTGTCAAGCTGACATGCACGCT GAGCTCCGGGCATTCATCATATGCCATCGCATGGCATCACCAACAA CCCGAGAAGGGACCACGATATCTCATGAAGCTAAACTCCGACGGA TCGCATTCGAAGGGGGATGGAATACCCGACCGATTTTCGGGATCAT CGAGCGGGGCGGAGAGATATTTGACGATCTCCTCTCTGCAAAGCG AGGACGAGGCGGACTACTATTGCCAAACCTGGGGCACGGGAATGC TATGGTTTGGAGGAGGCACACAACTGACGGCGCTGGGCCAACCG (SEQ ID NO. 114) |
| TE03 | GCGGCCGCCATGAATTTTGGACTGAGGCTGATTTTCCTGGTGCTGA CCCTGAAAGGCGTCCAGTGTCAAGTAGTCCTGACTCAAAGCCCCC CGGCGAGCGCATCATTGGGGGCGAGCGTCAAGCTGACATGCACG CTATCGAGCGGGCACTCTAGCTACGCGATAGCATGGCACCAACAA CAACCGGAAAAGGGACCCCGATACTTGATGAAATTAAATAGCGACG GATCGCACTCTAAGGGAGACGGAATACCTGATAGATTTAGCGGGA |
| | GCTCATCGGGGGCGGAGAGATACTTGACGATTAGCTCACTGCAAT CGGAGGATGAGGCGGACTACTATTGCCAAACATGGGGGACGGGAT GCTGCTTCGGAGGAGGCACGCAACTGACCGCATTGGGACAACCA (SEQ ID NO. 115) |
| TE04 | GCGGCCGCCATGAATTTTGGACTGAGGCTGATTTTCCTGGTGCTGA CCCTGAAAGGCGTCCAGTGTCAAACCGTGGTGACACAAGAATCAA GTTTTAGCGTATCGCCGGGAGGGACGGTGACGCTGACCTGCGGG CTATCATCTGGATCGGTATCAACATCCTACAATCCGAGCTGGTATC AACAAACGCCCGGACAAGCGCCACGAACCCTGATATATTCGACAAA TACCCGATCATCTGGGGTGCCGGATAGATTTTCCGGCTCGATTCTG GGAAACAAGGCTGCGCTGACGATAACCGGAGCTCAAGCCGACGAT GAGAGCGATTATTATTGCGTGCTATACATGGGGAGCGGGATTATAT TCGGATCTGGAACGAAAGTCACGGTGCTAGGACAACCG (SEQ ID NO. 116) |
| TE07 | GCGGCCGCCATGAATTTTGGACTGAGGCTGATTTTCCTGGTGCTGA CCCTGAAAGGCGTCCAGTGTCAAACGGTCGTCACGCAAGAGCCAT CCTTCTCGGTCTCGCCGGGGGGGACGGTCACACTGACATGCGGG CTGAGCTCGGGATCGGTCTCAACGAGCTACTACCCGAGCTGGTAT CAACAAACACCGGGGCAAGCACCGCGGACGCTGATATATTCCACA AATACACGGAGCTCCGGTGTCCCGGAGAGATTCTCGGGATCAATA CTGGGGAACAAGGCGGCTCTGACGATAACCGGAGCCCAAGCGGAT GACGAATCGGACTATTACTGCGTCCTATACATGGGCTCCGGCACAT GCTGCTTTGGAGGAGGCACACAACTGACGGCGCTGGGACAACCT (SEQ ID NO. 117) |
| TE10 | GCGGCCGCCATGAATTTTGGACTGAGGCTGATTTTCCTGGTGCTGA CCCTGAAAGGCGTCCAGTGTCAGCCGGTTTTGACGCAATCTAGCA GCGCATCCGCTAGTCTTGGAAGCTCCGTGAAGCTGACATGCACAC TATCATCGGGGCATTCCTCCTACATAATTGCATGGCATCAACAACAA CCCGGCAAGGCCCCGAGATACTTAATGAAACTGGAAGGATCGGGA TCATATAACAAAGGATCGGGGGTGCCGGATAGATTTAGCGGATCTA GCTCTGGGGCTGACCGATACCTGACGATCTCTAATCTGCAATTTGA GGACGAGGCCGATTACTACTGCGAAACATGGGATAGCAACACCCC ACACGCGGTATTTGGAGGAGGCACCCAATTGACCGCGCTAGGCCA ACCA (SEQ ID NO. 118) |
| TM1 | GTCATCCAGATGACCCAGTCTCCATCCTCCCTGTCTGCATCTGTAG GAGACAGAGTCACCATCACTTGCCGGGCAAGTCAGGGCATTAGAA ATGATTTAGGCTGGTATCTGCAGAAACCAGGGAAAGCCCCTGAGCT CCTGATCTATCCTGCATCCAGTTTACAAAGTGGGGTCCCGTCAAGG TTCAGCGGCAGTGGATCTGGCACAGATTTCACTCTCACCATCAGCA GCCTGCAGCCTGAAGATTTTGCAACTTATTACTGTCTACAAGATTAC AATTACCCGTTCACTTTTGGCCAGGGGACCAAGCTGGAGATCAAA (SEQ ID NO. 119) |

| **Table 11: Constant region amino acid sequences** | |
|---|---|
| **Constant region** | **AA** |
| Human IgG4 heavy chain P01861.1 | ASTKGPSVFPLAPCSRSTSESTAALGCLVKDYFPEPVTVSW NSGALTSGVHTFPAVLQSSGLYSLSSVVTVPSSSLGTKTYTC NVDHKPSNTKVDKRVESKYGPPCPPCPAPEFEGGPSVFLFP PKPKDTLMISRTPEVTCVVVDVSQEDPEVQFNWYVDGVEVH NAKTKPREEQFNSTYRVVSVLTVLHQDWLNGKEYKCKVSNK GLPSSIEKTISKAKGQPREPQVYTLPPSQEEMTKNQVSLTCL VKGFYPSDIAVEWESNGQPENNYKTTPPVLDSDGSFFLYSR LTVDKSRWQEGNVFSCSVMHEALHNHYTQKSLSLSLGK (SEQ ID NO. 120) |
| Human IgG4 heavy chain | ASTKGPSVFPLAPCSRSTSESTAALGCLVKDYFPEPVTVSW NSGALTSGVHTFPAVLQSSGLYSLSSVVTVPSSSLGTKTYTC NVDHKPSNTKVDKRVESKYGPPCPPCPAPEFEGGPSVFLFP PKPKDTLMISRTPEVTCVVVDVSQEDPEVQFNWYVDGVEVH NAKTKPREEQFNSTYRVVSVLTVLHQDWLNGKEYKCKVSNK GLPSSIEKTISKAKGQPREPQVYTLPPSQEEMTKNQVSLTCL VKGFYPSDIAVEWESNGQPENNYKTTPPVLDSDGSFFLYSR LTVDKSRWQEGNVFSCSVMHEALHNHYTQKSLSLSLG (SEQ ID NO. 180) |
| Human IgG2 heavy chain P01859 | ASTKGPSVFPLAPCSRSTSESTAALGCLVKDYFPEPVTVSW NSGALTSGVHTFPAVLQSSGLYSLSSVVTVPSSNFGTQTYT |
| | CNVDHKPSNTKVDKTVERKCCVECPPCPAPPVAGPSVFLFP PKPKDTLMISRTPEVTCVVVDVSHEDPEVQFNWYVDGVEVH NAKTKPREEQFNSTFRVVSVLTVVHQDWLNGKEYKCKVSN KGLPAPIEKTISKTKGQPREPQVYTLPPSREEMTKNQVSLTC LVKGFYPSDISVEWESNGQPENNYKTTPPMLDSDGSFFLYS KLTVDKSRWQQGNVFSCSVMHEALHNHYTQKSLSLSPGK (SEQ ID NO. 121) |
| Human light chain, lambda 1 P0CG04 | GQPKANPTVTLFPPSSEELQANKATLVCLISDFYPGAVTVAW KADGSPVKAGVETTKPSKQSNNKYAASSYLSLTPEQWKSH RSYSCQVTHEGSTVEKTVAPTECS (SEQ ID NO. 122) |
| Human light chain, lambda 2 P0DOY2 | GQPKAAPSVTLFPPSSEELQANKATLVCLISDFYPGAVTVAW KADSSPVKAGVETTTPSKQSNNKYAASSYLSLTPEQWKSHR SYSCQVTHEGSTVEKTVAPTECS (SEQ ID NO. 123) |
| Human light chain, kappa P01834 |  (SEQ ID NO. 124) |

### EXAMPLES

### Material and methods

### In vivo experimental design

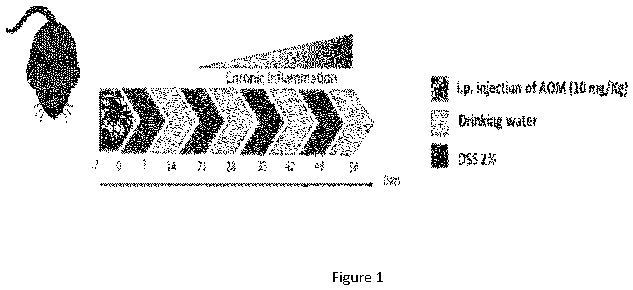

As shown in **Figure 1****,** after a single intraperitoneal injection of AOM (10 mg/kg body weight in physiological solution), mice were treated with repeated oral cycles of 2% (weight/volume) DSS, molecular mass, 40 kDa (MP Biomedicals), each characterized by 7 days of DSS exposure in drinking water, followed by 7 days of regular drinking water, according to the scheme of **Figure 1** (12).

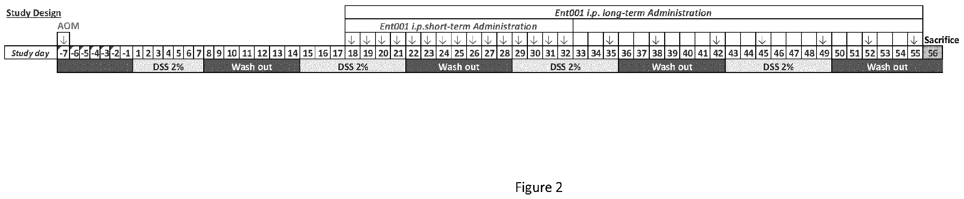

The design of the study is summarized in **Figure 2****.**

Ent001 is the antibody described herein and in WO2021094620 as TC01.

25 Days after AOM administration and 18 Days after starting DSS administration, mice have been treated with testing drugs through intraperitoneal injections according to the following treatment groups:
1) Vehicle (N=8 mice)
2) Ent001 alone (no AOM/no DSS) (N=8 mice)
3) AOM + Ent001 long-term treatment (N=8 mice)
4) DSS + Ent001 long-term treatment AOM/DSS + Vehicle (N=8 mice)
5) AOM/DSS + Ent001 short-term treatment (N=8 mice)
6) AOM/DSS + Ent001 long-term treatment (N=8 mice)
7) AOM/DSS + anti-murine TNFalpha long-term treatment (N=8 mice)
8) AOM/DSS + anti-murine TNFalpha long-term treatment + Ent001 long-term treatment (N=8 mice)

Anti-murine TNFalpha was used as positive control.

Of note, the commercial clone MP6-XT22 (eBioscience, San Diego, CA) was used, since it displayed beneficial effects, at the indicated dose, in the DSS-induced model of colitis, as documented by the literature (13).

### Clinical and Physical Examination: Disease Activity Index & Body Weight Loss

Colitis severity have been monitored using a disease activity index (DAI) score based on daily evaluation of body weight, stool consistency, and presence of blood in the stools; grading of intestinal inflammation has been determined according to the criteria proposed by Cooper et al. (14, 15); the DAI was determined by scoring changes in: weight loss (0 = none, 1 = 1%-5%, 2 = 5%-10%, 3 = 10%-20%, 4 = >20%); stool consistency (0 = normal, 2 = loose, 4 = diarrhoea); and rectal bleeding (0 = normal, 2 = occult bleeding, 4 = gross bleeding). A 5-point (0-4) DAI was thus obtained as the mean of the three parameter scores.

### Tissue sampling

At Day 56, mice have been euthanized by CO₂, colons excised (without caecum), and collected to perform the following analyses:
- **Measurement of colon length,** as an additional parameter of intestinal inflammation.
- **Histological analysis:** whole colons have been fixed in formalin and embedded in paraffin, in order to perform histologic inflammatory scores and evaluation of tumor density, grade and size.
- **Quantification of intestinal epithelial cell proliferation:** mice (n=4 per group) have been administered with 5-Bromo-2'-deoxyuridine (BrdU, from Merck Life Science S.r.l.) by intraperitoneal injection, 1 hour before sacrifice. Formalin-fixed and paraffin-embedded sections have been stained using an anti-BrdU antibody and BrdU-positive nuclei have been quantified using an imaging system.
- **Quantification of cleaved Caspase 8:** as an additional redout to confirm the efficient inhibition of IGFBP3, we have quantified IGFBP3-dependent apoptotic molecule cleaved Caspase 8 in the intestinal mucosa of n=4 animals per group, by western blot.

### Assessment of histological colonic lesions

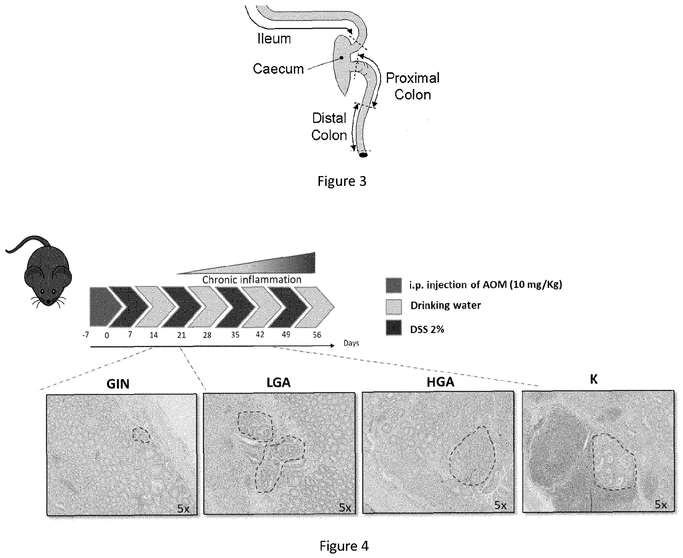

Grading of intestinal inflammation was confirmed histologically. At sacrifice, colons were fixed in 10% formalin for 24-48 hour at room temperature. Tissues were then dehydrated through 70%, 80%, and 95% alcohol, 45 min each, followed by 3 changes of 100% alcohol, 1 hour each. Colons were then cleared through 2 changes of xylene, 1 hour each, and then immersed in 3 changes of paraffin, 1 hour each. 2-µM paraffin-embedded sections were obtained using a microtome and transferred onto glass slides suitable for immunohistochemistry (Superfrost Plus). Upon staining with hematoxylin (Dako) and eosin (Diapath), a blinded pathologist evaluated the degree of inflammatory cell infiltration and mucosal damage on colon sections, using 2 different protocols:
1) Rachimilewitz score (16). This scoring system takes into account the ulceration, extent of ulceration, flogosis, extent of flogosis and fibrosis in the whole colon section, without distinguishing into proximal and distal colon. Minimal score was 0, and maximal score was 20, as a sum of all single scores (Total histologic score).
2) Histologic score, as described by Dieleman et al. (17). The sections were graded in the proximal and distal colons **(****Figure 3****)** by a blinded pathologist with a range from 0 to 3 as to amount of inflammation, depth of inflammation and with a range from 0 to 4 as to the amount of crypt damage or regeneration as indicated in **Table 12.** These changes were also quantified as to the percentage involvement by the disease process: (1) 1-25%; (2) 26-50%; (3) 51-75%; (4) 76-100%. Each section was then scored for each feature separately by establishing the product of the grade for that feature and the percentage involvement (in a range from 0 to 12 for inflammation and for extent, and in a range from 0 to 16 for regeneration and for crypt damage). Total histological score was calculated as the sum of the score products (in a range from 0 to 56).

40 kDa DSS-induced Inflammation model, as the one used in this study, affords a high degree of uniformity and reproducibility of most lesions in the distal colon, where an enormous number of microorganisms live, whereas less inflammation is observed in the proximal site and almost none in the caecum. On the contrary, mice treated with 5 kDa DSS develop relatively patchy lesions mainly in the cecum and upper colon (18-20).

**Table 12. Histologic score by Dieleman et al.**

| **Dieleman et al.** | | |
|---|---|---|
| Inflammation | 0 | None |
| | 1 | Slight |
| | 2 | Moderate |
| | 3 | Severe |
| | | |
| Extent | 0 | None |
| | 1 | Mucosa |
| | 2 | Mucosa and submucosa |
| | 3 | Transmural |
| | | |
| Regeneration | 0 | Complete regeneration or normal tissue |
| | 1 | Almost complete regeneration |
| | 2 | Regeneration with crypt depletion |
| | 3 | Surface epithelium not intact |
| | 4 | No tissue repair |
| | | |
| Crypt damage | 0 | None |
| | 1 | Basal 1/3 damaged |
| | 2 | Basal 2/3 damaged |
| | 3 | Only surface epithelium intact |
| | 4 | Entire crypt and epithelium lost |
| | | |
| Percent involvement | 1 | 1-25% |
| | 2 | 26-50% |
| | 3 | 51-75% |
| | 4 | 76-100% |

### Analysis and grading of tumor lesions

Colons of animals that have been treated with AOM/DSS are found to have occasional, abnormally large, darkly staining, and slightly raised 'aberrant crypts', called glandular intraepithelial neoplasia (GIN), after 3-4 weeks from the AOM injection (around Day 14 to Day 21 of the present study). With longer time intervals between carcinogen administration and inflammation, these aberrant crypts are frequently found as foci, with two to many hundreds of aberrant crypts appearing together in a cluster, after 5-7 weeks (around Day 28 to Day 42 of the present study) from AOM injection. Many aberrant crypts on histological examination show a low and a high level of dysplasia and are then correctly called adenoma that may evolve to non-metastatic colon carcinomas at 7-8 weeks (around Day 42 to Day 56 of the present study) (**Figure 4**). Thus, four grades of mucosal lesions have been identified and quantified by a blinded pathologist: glandular intraepithelial neoplasia (GIN), indicating small clusters of dysplastic colonic crypts on the mucosal surface, low grade (LG) and high-grade (HG) adenomas, and *in situ* carcinoma (K) (12, 21). Mice were sacrificed at Day 56, seven days after the fourth DSS cycle, and during the recovery phase in water. This was done because around this time point not only there are higher chances to find different grades of tumoral lesions, including in situ carcinoma, but also because the reduced inflammation during recovery allows a better visualization and quantification of neoplastic lesions.

### BrdU quantification on colon sections

To achieve labelling of epithelial cells undergoing DNA synthesis, mice (n=4 per group) received an i.p. injection of 100 mg/kg BrdU 1 hour prior to sacrifice. Formalin-fixed, paraffin-embedded colon sections (2-µm) were stained for BrdU after antigen retrieval for 30 minutes at 98°C in citric buffer (10 mM, pH 6.0). Following endogenous peroxidase blocking with peroxidase 1 solution for 20 minutes at RT, tissues were washed in TBS. Upon incubation with the rat anti-BrdU Ab (1:600; AbD Serotec) for 40 minutes at 4°C, a rat HRP polymer kit (Biocare Medical) was used to detect BrdU using DAB (Biocare Medical) as chromogen. Tissue sections were then counterstained with CAT Hematoxylin (Biocare Medical), dried overnight in a 37°C oven, and mounted in Eukitt Quick Hardening Mounting Media. Images were acquired with a VS120 Dot Slide System (Olympus), and BrdU+ cells were counted in consecutive and randomly selected 10x fields (n = 4 fields of 1 mm²/section) using the IHC Tool Box of ImageJ 1.80p (see examples below). We distinguished inflammatory from tumor areas.

Non-inflammatory (control groups) and inflammatory areas (colitic and tumor-bearing mice): upon definition of the region of interest (ROI) (1 mm²), containing all mucosa layers (whenever possible), BrdU-positive nuclei with different sizes were selected using the ellipse tool, through the TRAINING-NUCLEI function. Image J automatically processes the image and calculates the number of nuclei within the selected region. Tumor areas: in this case nuclei segmentation and quantification has been performed in selected tumor areas calculated in pixels on a 20x magnification, and results are reported as number of BrdU-positive nuclei per tumor area.

### Quantification of cleaved Caspase 8 by western blot

Colonic samples (1 entire colon from 1 mouse per group + 1 small specimens from the proximal colon of 3 mice per group) were mechanically homogenized in lysis buffer (Tris 50 mM, 2% SDS, 1 mM Na₂VO₂) containing proteinase inhibitors (Complete Mini, Roche Diagnostics GmbH, Penzberg, Germany), for protein extraction. Insoluble material was removed by centrifugation for 30 min at 14,000 rpm at 4°C. The concentration of proteins in each lysate was measured using the Bio-Rad protein assay (Bio-Rad Laboratories).

Next, proteins from each sample were separated on a 10% Tris-glycine polyacrylamide gel at 160 V for 80 minutes and blotted onto PVDF-membranes (Millipore, Schwalbach, Germany) at 80 mA for 90 min. Membrane was cut according to protein weight and nonspecific binding was blocked with Tris-buffered saline (TBS) containing 5% non-fat dried milk and 0.1% Tween 20, followed by overnight incubation at 4°C with the rabbit anti-mouse Caspase 8 antibody which recognizes the active/cleaved form of Caspase 8 antibody (NB100-56116 from Novus Biological); 1:2000), and with a rabbit anti Caspase-8 antibody (Cell Signaling Technology, #4927; 1:500). Membranes were washed for 1 h with TBS containing 0.1% Tween 20 and then incubated for 1 h with the appropriate horseradish peroxidase-conjugated secondary antibody (1:3000; GE Healthcare). The membranes were then incubated with Immobilon Western Chemilum (Millipore) for 1-5 minutes, after which bands were detected by Chemidoc (BioRad Laboratories), using Quantity One software. The filter was then stripped with buffer Restore (Pierce) and reprobed with a mouse anti-actin antibody (clone C11; 1:1000; Santa Cruz Biotechnology) for control protein loading. Protein bands were quantified using the densitometry program Image J.

### Statistical analysis

Statistical analyses were performed using GraphPad Prism 7 (GraphPad Software). Data are presented as mean ± Standard Error of Mean (SEM) and differences were considered statistically significant when P < 0.05. For experiments including more groups a one-way or two-way ANOVA multivariate analysis has been performed accompanied by a post-hoc modification test.

### RESULTS

### Example 1

### Clinical and Physical Examinations: Disease Activity Index & Body Weight Loss

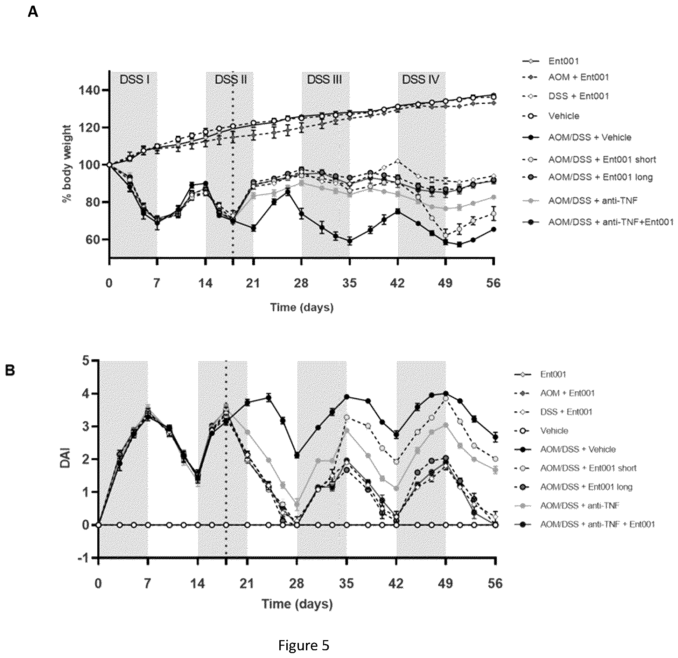

After 2 weeks of treatments (Day 18-33, treatment every day), results show that Ent001 significantly inhibits experimental colitis in comparison with control group (AOM/DSS + vehicle), in terms of percentage of body weight loss **(****Figure 5A****)** and DAI **(****Figure 5B****).** Statistical analyses and p values of specific groups at each time point can be found in following **Tables 13** and **14.**

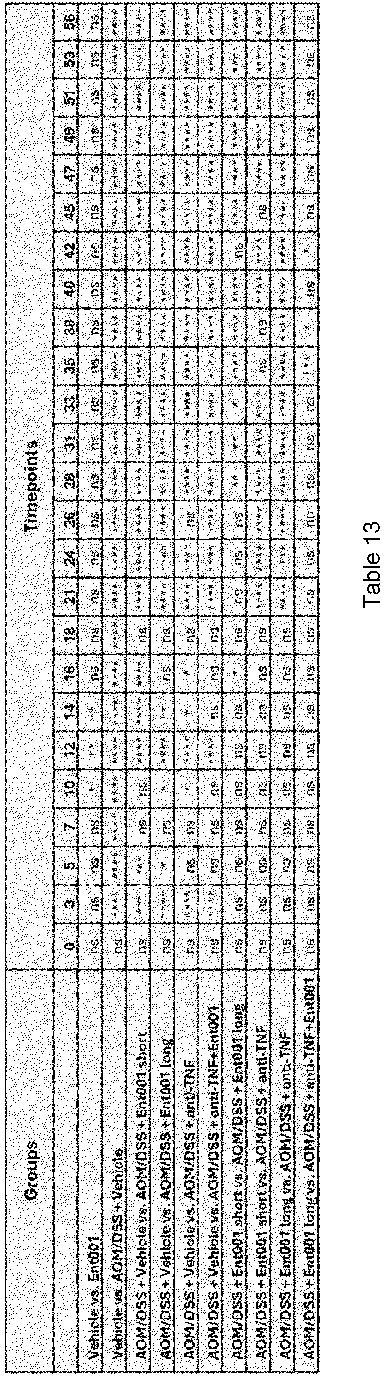

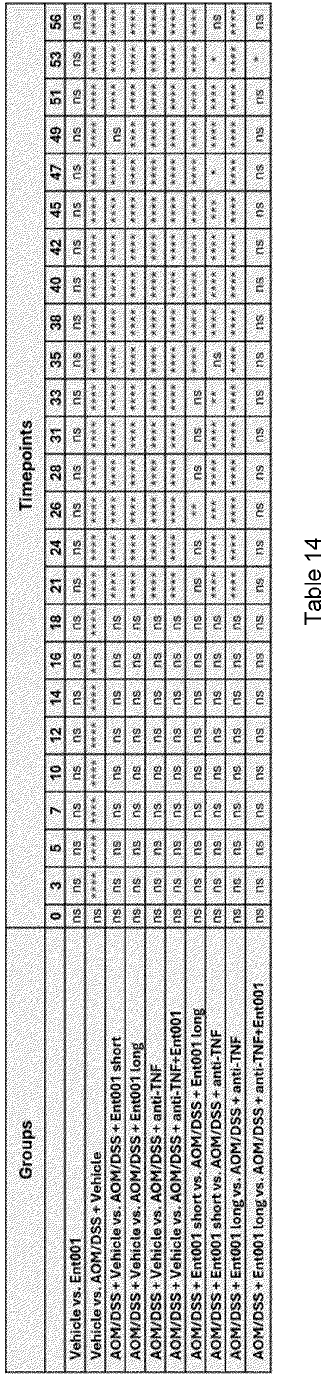

Of note, Ent001 was significantly more efficient than anti-murine TNFalpha in reducing body weight loss and DAI, at all-time points between Day 18 and Day 33. Combination of anti-murine TNFalpha + Ent001 was as efficient as Ent001 alone.

In the following weeks (treatment 2 times per week) results confirmed the statistically significant inhibitory effect of Ent001 on experimental colitis also from Day 33 to Day 56, in comparison with control group (AOM/DSS + vehicle), in terms of body weight loss **(****Figure 5A****)** and DAI **(****Figure 5B****).** Again, Ent001 was significantly more efficient than anti-murine TNFalpha in reducing both these clinical parameters of inflammation, whereas combination of anti-murine TNFalpha + Ent001 was as efficient as Ent001 alone.

Of note, in the AOM/DSS + Ent001 short-treatment (short) group mice developed severe colitis from Day 44 to Day 56, as in the control group (AOM/DSS + vehicle). Differences were still statistically significant between AOM/DSS + Ent001 short-treatment vs AOM/DSS + vehicle, but this may be due to the reduced inflammatory conditions in the AOM/DSS + Ent001 short-treatment (short) group at Day 33, when administration of the antibody was interrupted.

Interestingly, in the AOM/DSS + Ent001 short-treatment group, anti-inflammatory effects remained at high levels until Day 44 even in the absence of the antibody, and particularly in terms of percentage of body weight loss, suggesting that the beneficial effects of Ent001 may last for at least 1 week after the end of treatment.

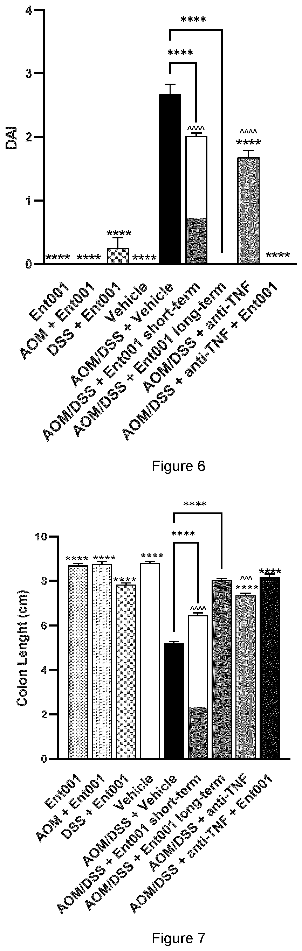

As shown in **Figure 6****,** at sacrifice (Day 56), all treatment groups showed a significantly lower DAI than AOM/DSS + vehicle group. Of note, long-term treatment with Ent001 resulted in a much better control on DAI compared to anti-murine TNFalpha (anti-TNF).

### Example 2

### Post-mortem analysis of colon length

Colon shortening is a parameter of intestinal inflammation (20). At Day 56 mice were sacrificed and colon lengths were measured.

Results confirmed the statistically significant inhibitory effect of Ent001 on the severity of colitis in comparison with control group (AOM/DSS + vehicle) **(****Figure 7****).** Ent001, when administered from Day 18 to Day 55 (i.e. long-term), was significantly more efficient than anti-murine TNFalpha in reducing colon shortening, and combination of anti-murine TNFalpha + Ent001 was as efficient as Ent001 alone.

Differences were still statistically significant between AOM/DSS + Ent001 short-term vs AOM/DSS + vehicle, but this again may be due to the reduced inflammatory conditions in the AOM/DSS + Ent001 short-term group at Day 33, when administration of Ent001 was interrupted. Nevertheless, differences between AOM/DSS + Ent001 short-term and AOM/DSS + Ent001 long-term were statistically significant.

### Example 3

### Evaluation of colonic inflammation at the histological level

The protective effects of Ent001 in DSS-induced chronic colitis were confirmed by a blinded pathologist using the histologic score by Rachmilewitz et al (16) and Dieleman et al (17).

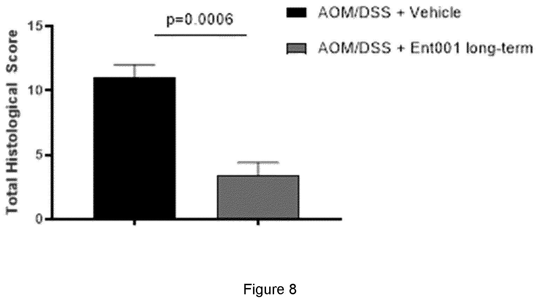

According to the Rachmilewitz score, performed on the whole colon, results show that Ent001 significantly improved colitis **(****Figure 8****)** compared to control group (AOM/DSS + vehicle) in terms of total histological score.

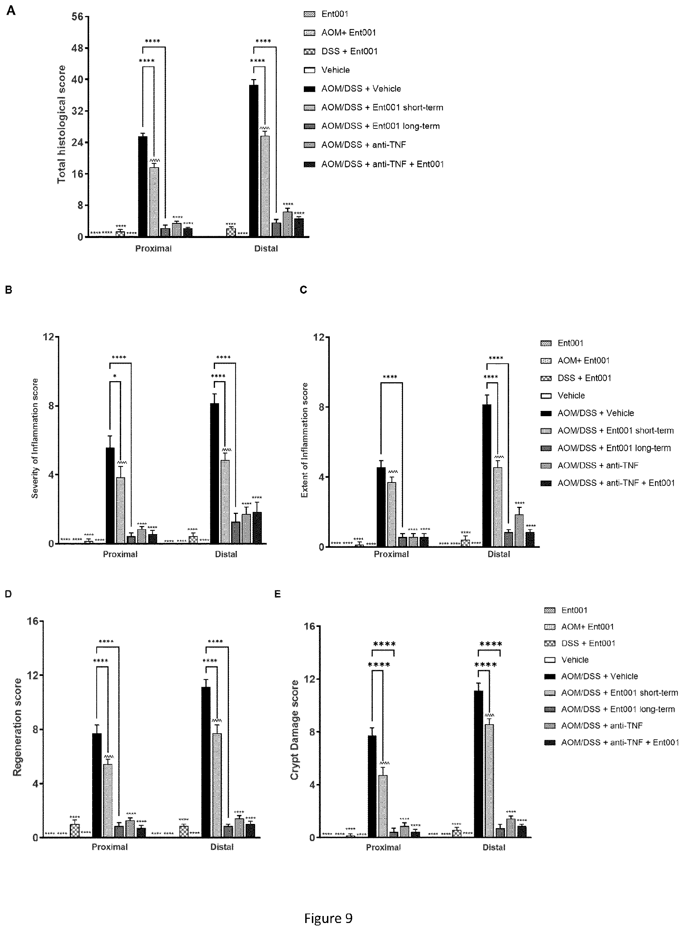

According to the Dieleman score, performed separately on the proximal and distal colon, results show that Ent001 significantly inhibits experimental colitis in comparison with control group (AOM/DSS + vehicle), in terms of total histologic score **(****Figure 9A****),** severity of inflammation **(****Figure 9B****),** extent of inflammation **(****Figure 9C****),** regeneration **(****Figure 9D****)** and crypt damage **(****Figure 9E****).** In both proximal and distal colons there are no statistically significant differences between groups treated with Ent001 and anti-murine TNFalpha. Of note, Ent001 short-term treatment group was significantly different when compared to the control group (AOM/DSS + vehicle), for all clinical histological parameters.

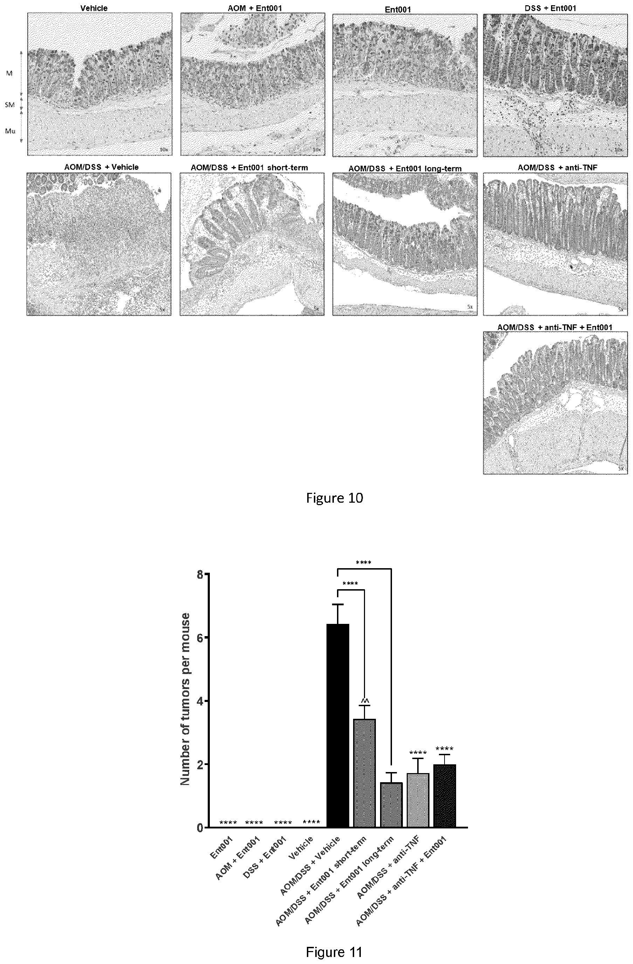

Representative images of inflammatory conditions per group are shown in **Figure 10****.** In healthy mice (e.g. Vehicle) the tissue layers (mucosa, muscularis mucosae, submucosa and muscularis propria) are well structured. Administrations of AOM + Ent001, or Ent001 alone do not affect the layer composition and there is no inflammatory infiltrate. In DSS-induced colitic mice (e.g. AOM/DSS + vehicle) layer stratification is not maintained anymore, a massive inflammatory infiltrate completely occupies both mucosa and submucosa, and crypt are severely damaged in most part of the colon. The epithelium is eroded indicating the presence of ulcerations.

Treatment with Ent001 significantly ameliorates experimental colitis: tissue layers are maintained, there is a slight inflammation with inflammatory cells infiltrating both mucosa and submucosa, but both epithelium and crypts are well structured. At the histological level we observed no major differences between groups treated with Ent001 or anti-murine TNFalpha or the combination of Ent001 and anti-murine TNFalpha. In the AOM/DSS + Ent001 short-term treatment group, we observed a moderate-to severe inflammation, with partially restored crypts and epithelium, but with a pronounced flogosis; nevertheless, when compared with the AOM/DSS vehicle group differences were still significant. As stated above, this may be due to the reduced inflammatory conditions in the AOM/DSS + Ent001 short-term treatment group at Day 33, when administration of the antibody was interrupted.

### Example 4

### Analysis & grading of tumor lesions

The objective of this study was to identify a potential effect of modulation, in terms of tumor initiation and promotion, tumor growth or tumor protection in the AOM/DSS mouse model of colitis-associated cancer.

When we looked at the total tumor density, i.e. total number of tumors/mouse, we found no tumors in the vehicle (as expected), Ent001, AOM + Ent001 and DSS + Ent001 groups, indicating that Ent001 antibody does not induce nor accelerate tumor initiation in the colon **(****Figure 11****).** In AOM/DSS treated mice, we observed a certain number of tumors per mouse, **that were significantly reduced by treatment with Ent001** and/or anti-murine TNFalpha. Notably, in the AOM/DSS + Ent001 short-term group, tumor density was higher than other groups treated with Ent001 long-term but still significantly lower than the control AOM/DSS + vehicle group, indicating that the moderate degree of inflammation between Day 33 and 56 may have accelerated tumor promotion, but not enough to reach tumor density levels of AOM/DSS + vehicle.

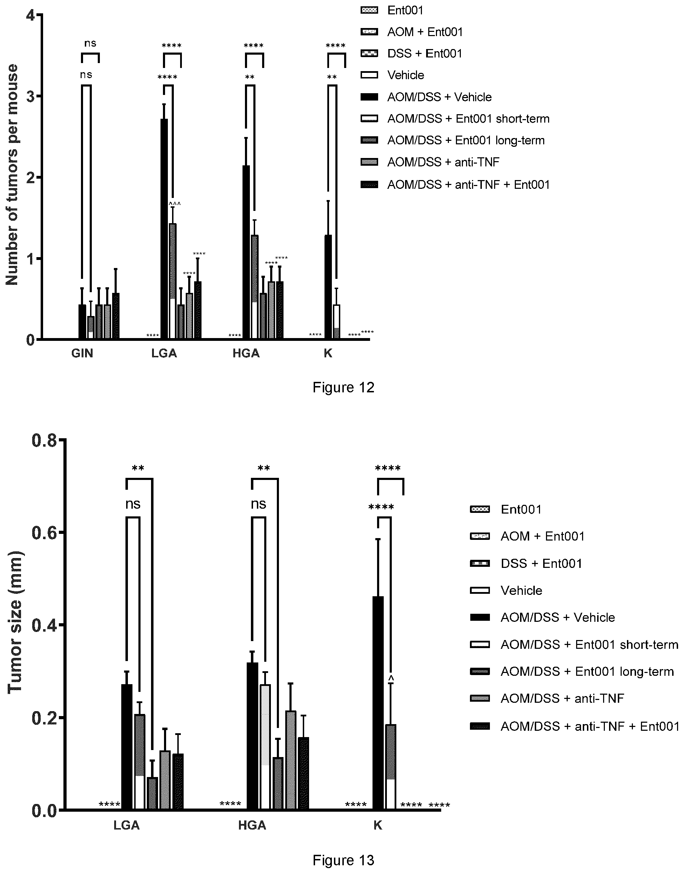

In terms of tumor grade, we observed no significative differences in GIN density among the various groups **(****Figure 12****).** This is not surprising, since GIN are neoplastic lesions that develop around 3-4 weeks after AOM injection, when we just started to treat mice with Ent001 and/or anti-murine TNFalpha. On the contrary we observed a significative reduction in the density of LGA and HGA in tumor-bearing mice treated with Ent001 and/or anti-murine TNFalpha in comparison with AOM/DSS + vehicle control group **(****Figure 12****),** including the AOM/DSS + Ent001 short-term group.

Of note, progression from adenoma (LGA/HGA) to carcinoma (K) was observed only for the AOM/DSS + vehicle and the AOM/DSS + Ent001 short-term groups with a significantly higher number in the control group **(****Figure 12****),** suggesting that Ent001, blocks AOM/DSS-induced tumor progression.

When we looked at the tumor size, we found significative reduction in the AOM/DSS + Ent001-long term group when compared to AOM/DSS + vehicle, both for LGA and HGA **(****Figure 13****),** and a trend for the other groups. Carcinomas (K) were significantly smaller in the AOM/DSS + Ent001 short-term group versus control. These results could be due to a direct effect of Ent001 antibody on intestinal epithelial biologic functions (e.g. proliferation), whereas anti-murine TNFalpha acts only indirectly on tumor growth by modulating inflammation.

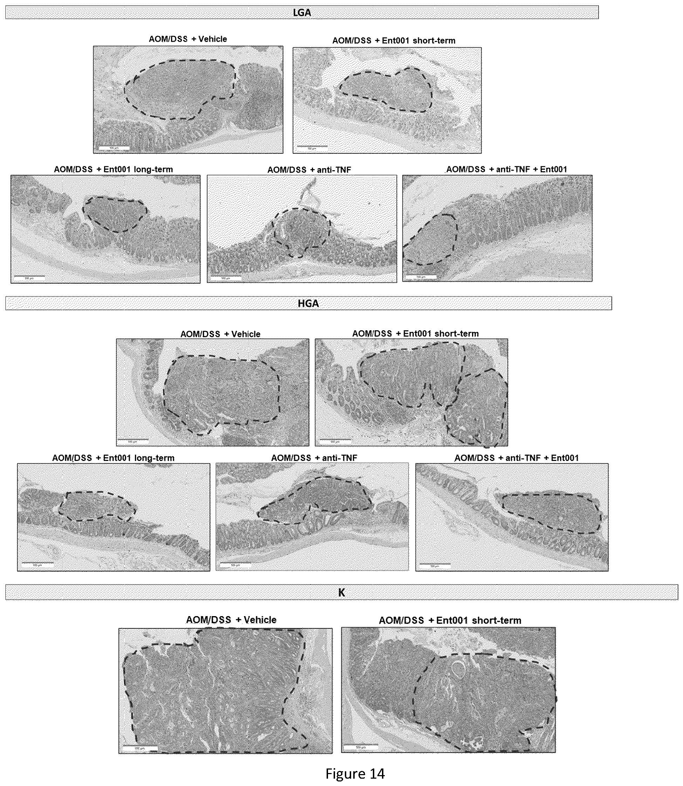

Representative images of tumor size variation in LGA, HGA and K tumors are shown in **Figure 14****.**

### Example 5

### BrdU quantification on colon sections

In order to evaluate the impact of Ent001 on intestinal epithelial cell proliferation, mice (n=4 per group) received an i.p. injection of 100 mg/kg BrdU 1 hour prior to sacrifice, time that allows incorporation of BrdU only by proliferating intestinal epithelium and not by other cell type with slower cell cycle (e.g. leukocytes). Formalin-fixed, paraffin-embedded colon sections were stained using an anti-BrdU antibody. BrdU-positive nuclei were quantified in non-inflammatory (control groups), inflammatory (colitic and tumor-bearing mice) and tumor areas, as described above. In inflammatory areas of AOM/DSS mice, the number of BrdU-positive nuclei is significantly increased compared to Vehicle group, due to accelerated epithelial cell turnover caused by chronic inflammation in combination with DNA damage caused by AOM (22).

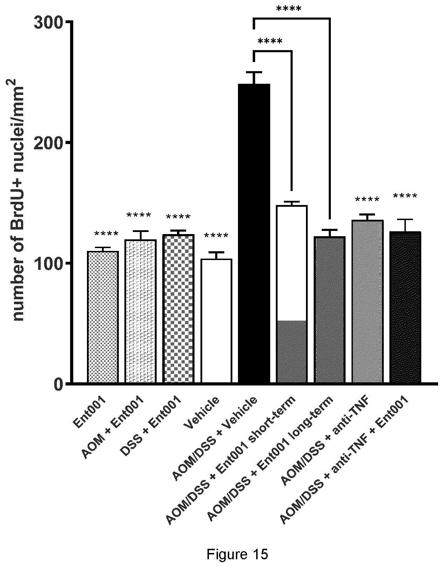

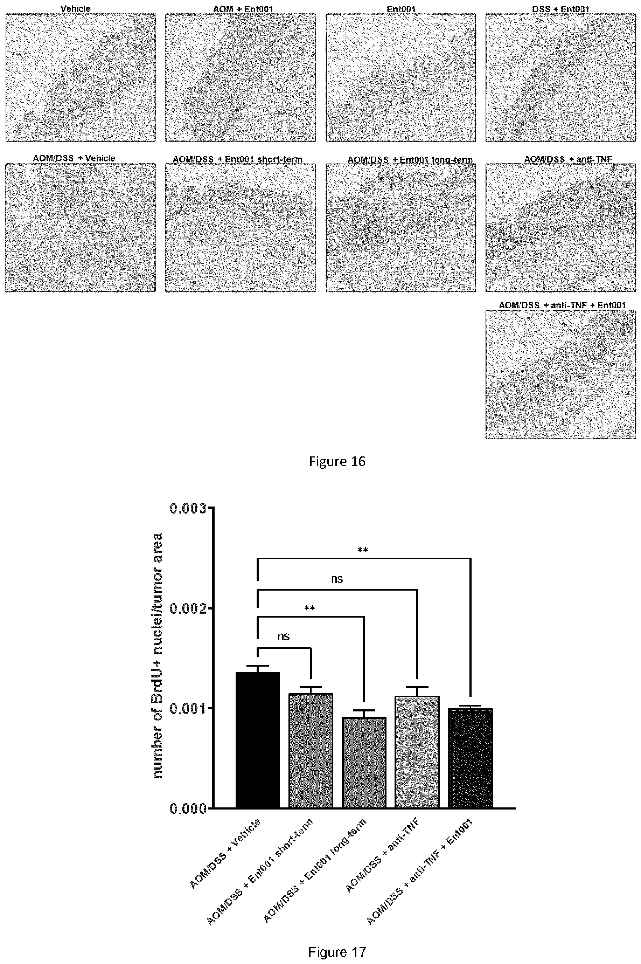

In both non-inflammatory and inflammatory regions, results showed that treatment with Ent001 alone in the presence or absence of AOM, or in DSS-induced colitic mice does not affect epithelial cell proliferation **(****Figure 15** and **16****).** In AOM/DSS mice treatment with Ent001 and anti-murine TNFalpha significantly reduced the number of BrdU-positive nuclei/mm² when compared with AOM/DSS + vehicle, including the AOM/DSS + Ent001 short group.

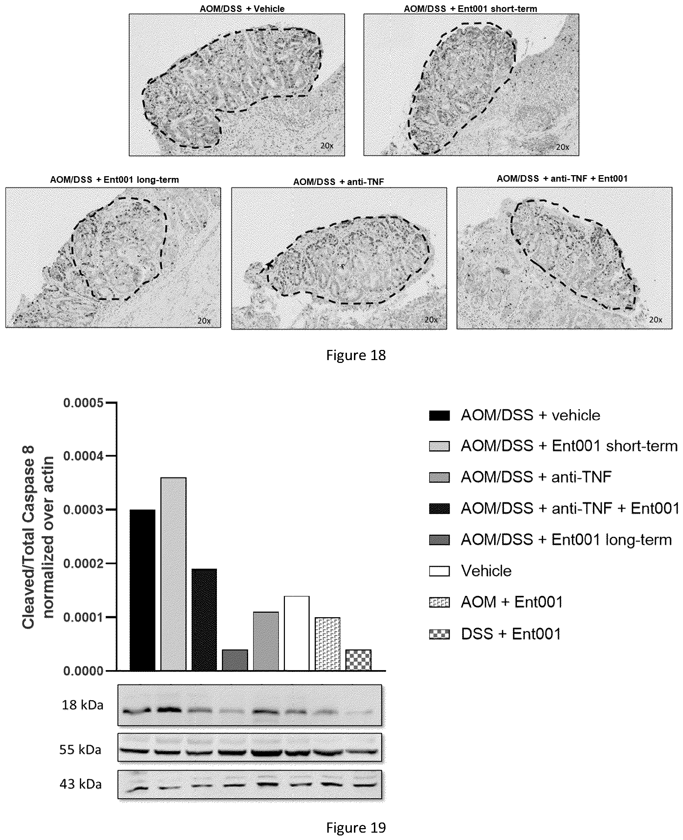

Within tumors (i.e. LGA), treatment with Ent001 significantly reduced the number of BrdU-positive nuclei per tumor area, while AOM/DSS + anti-murine TNFalpha and AOM/DSS + Ent001 short-term group did not **(****Figure 17** and **18****).** This data indicates that Ent001 might impact intestinal epithelial cell proliferation.

### Example 6

### Quantification of cleaved Caspase 8

In order to confirm the efficient inhibition of IGFBP3-dependent Caspase 8 activation by Ent001, we have quantified cleaved Caspase 8 and total Caspase 8 protein in the colon lysates from n=4 animals per group (1 whole colon + 3 small proximal pieces from 3 mice), by western blot. Lysates from the same group were pooled to increase the total amount of proteins. Upon normalization with the loading control (actin), results showed that the ratio Cleaved Caspase 8/total Caspase 8 is reduced in mice treated with Ent001 **(****Figure 19****),** confirming efficacy for Ent001.

### Conclusions

This study showed that, in the AOM/DSS model of colon carcinogenesis, not only Ent001 does not induce or accelerate tumor initiation in the colon, but also, significantly reduces tumor density, size and progression.

A potential synergist or antagonistic effect of Ent001 with anti-murine TNFalpha on tumor modulation was also evaluated and results indicated that the combination of anti-murine TNFalpha + Ent001 is as efficient as Ent001 alone.

These data support TMEM219 as a potential therapeutic target for the treatment of colon cancer, in particular of colitis-associated cancer.

### REFERENCES

1. Sung, H., et al., Global cancer statistics 2020: GLOBOCAN estimates of incidence and mortality worldwide for 36 cancers in 185 countries. CA Cancer J Clin. 2021; 71: 209-249. https://doi.org/10.3322/caac.21660
2. Sawicki, T., et al., A review of colorectal cancer in terms of epidemiology, risk factors, development, symptoms and diagnosis. Cancers. 2021; 13, 2025. https://doi.org/10.3390/cancers13092025
3. Brenner, H., Kloor, M., and Pox, C. P., Colorectal cancer. The Lancet. 2014; 383(9927): 1490-1502. https://doi.org/10.1016/30140-6736(13)61649-9
4. Siegel, R.L., et al., Colorectal cancer statistics, 2023. CA Cancer J Clin. 2023; 73(3): 233-254. doi:10.3322/caac.21772
5. Dan, W., et al., Update and latest advances in mechanisms and management of colitis-associated colorectal cancer. World J Gastrointest Oncol. 2023 August 15; 15(8): 1317-1331. doi: 10.4251/wjgo.v15.i8.1317
6. Schmitt, M., and Greten, F., R., The inflammatory pathogenesis of colorectal cancer. Nat Rev Immunol. 2021 Oct;21(10):653-667. doi: 10.1038/s41577-021-00534-x
7. PDQ® Adult Treatment Editorial Board. PDQ Colon Cancer Treatment. Bethesda, MD: National Cancer Institute. Available at: https://www.cancer.gov/types/colorectal/hp/colon-treatment-pdq. Accessed <01/23/2024>. [PMID: 26389297]
8. Xie, YH., Chen, YX. & Fang, JY. Comprehensive review of targeted therapy for colorectal cancer. Sig Transduct Target Ther 5, 22 (2020). doi:10.1038/s41392-020-0116-z
9. Van K. Morris et al., Treatment of Metastatic Colorectal Cancer: ASCO Guideline. JCO 41, 678-700(2023). doi:10.1200/JCO.22.01690
10. Lu, J., et al., Thalidomide Attenuates Colitis and Is Associated with the Suppression of M1 Macrophage Polarization by Targeting the Transcription Factor IRF5. Dig Dis Sci 2021; 66: 3803-3812. doi:10.1007/s10620-021-07067-2
11. Zhou, R.W., Harpaz, N., Itzkowitz, S.H. et al. Molecular mechanisms in colitis-associated colorectal cancer. Oncogenesis 12, 48 (2023). https://doi.org/10.1038/s41389-023-00492-0
12. Fazio V, Robertis M, Massi E, et al. The AOM/DSS murine model for the study of colon carcinogenesis: From pathways to diagnosis and therapy studies. J Carcinog 2011; 10: 9. DOI: 10.4103/1477-3163.78279
13.Xiao Y-T, Yan W-H, Cao Y, et al. Neutralization of IL-6 and TNF-α ameliorates intestinal permeability in DSS-induced colitis. Cytokine 2016; 83: 189-192.
14. Cooper HS, Murthy SN, Shah RS, et al. Clinicopathologic study of dextran sulfate sodium experimental murine colitis. Lab Invest 1993; 69: 238-49. PMID: 8350599
15. D'Alessio S, Correale C, Tacconi C, et al. VEGF-C-dependent stimulation of lymphatic function ameliorates experimental inflammatory bowel disease. J Clin Invest 2014; 124: 3863-3878. DOI: 10.1172/JCI72189
16. Rachmilewitz D, Karmeli F, Takabayashi K, et al. Immunostimulatory DNA ameliorates experimental and spontaneous murine colitis. Gastroenterology 2002; 122: 1428-1441.
17. Dieleman, Palmen, Akol, et al. Chronic experimental colitis induced by dextran sulphate sodium (DSS) is characterized by Th1 and Th2 cytokines. Clin Exp Immunol 1998; 114: 385-391. DOI: 10.1046/j.1365-2249.1998.00728.x
18. Perše M, Cerar A. Dextran Sodium Sulphate Colitis Mouse Model: Traps and Tricks. J Biomed Biotechnol 2012; 2012: 1-13. DOI: 10.1155/2012/718617
19. KITAJIMA S, TAKUMA S, MORIMOTO M. Histological Analysis of Murine Colitis Induced by Dextran Sulfate Sodium of Different Molecular Weights. Exp Anim 2000; 49: 9-15. DOI: 10.1538/expanim.49.9
20.Chassaing B, Aitken JD, Malleshappa M, et al. Dextran Sulfate Sodium (DSS)-Induced Colitis in Mice. Curr Protoc Immunol; 104. Epub ahead of print 4 February 2014. DOI: 10.1002/0471142735.im1525s104.
21.Tacconi C, Ungaro F, Correale C, et al. Activation of the VEGFC/VEGFR3 Pathway Induces Tumor Immune Escape in Colorectal Cancer. Cancer Res 2019; 79: 4196-4210. DOI: 10.1158/0008-5472.CAN-18-3657
22. Grazioso TP, Brandt M, Djouder N. Diet, Microbiota, and Colorectal Cancer. iScience. 2019; 21:168-187. doi: 10.1016/j.isci.2019.10.011.
23. D'Addio F, et al., Circulating IGF-I and IGFBP3 Levels Control Human Colonic Stem Cell Function and Are Disrupted in Diabetic Enteropathy. Cell stem cell. 2015;17(4):486-98.
24.Jung P, et al., Isolation and in vitro expansion of human colonic stem cells. Nature Medicine 2011;17:1225-1227.
25. Huch M, et al., In vitro expansion of single Lgr5+ liver stem cells induced by Wnt-driven regeneration. Nature 2013; 494 (7436): 247-250
26. Mahé MM, et al., Establishment of Gastrointestinal Epithelial Organoids. Curr Protoc Mouse Biol 2013; 3: 217-240
27. Beck A, et al., Strategies and challenges for the next generation of therapeutic antibodies. Nat Rev Imm 2010; 10: 345-352

## Claims

1. An antibody that binds to TMEM219 receptor or an antigen binding fragment thereof for use in the prevention and/or treatment of colorectal cancer.

2. The antibody for the use according to claim 1 wherein said antibody inhibits or reduces the binding of IGFBP3 to said TMEM219 receptor and/or inhibits, reduces, or neutralizes the activation of the TMEM219 receptor induced by binding of IGFBP3 and/or does not activate TMEM219 pathway upon binding to TMEM219 receptor.

3. The antibody for the use according to claim 1 or 2 wherein said colorectal cancer is selected from the group consisting of colon cancer, bowel cancer, and rectal cancer.

4. The antibody for the use according to any one of claims 1-3 wherein said cancer is colitis-associated colorectal cancer.

5. The antibody for the use according to any one of claims 1-4 wherein said antibody comprises:
a. a heavy chain variable domain (VH) comprising:
i. a CDR1 sequence of the amino acid sequence selected from the group consisting of: SEQ ID NO: 8, 1, 10, 56, 59, 62, 65 and 68;
ii. a CDR2 sequence of the amino acid sequence selected from the group consisting of: SEQ ID NO: 5, 2, 11, 57, 60, 63, 66 and 69; and
iii. a CDR3 sequence of the amino acid sequence selected from the group consisting of: SEQ ID NO:6, 3, 7, 9, 12, 13, 58, 61, 64, 67 and 70; and/or
b. a light chain variable domain (VL) comprising:
i. a CDR1 sequence of the amino acid sequence selected from the group consisting of: SEQ ID NO:17, 14, 20, 23, 26, 29, 71, 77, 80, 82 and 85;
ii. a CDR2 sequence of the amino acid sequence selected from the group consisting of: SEQ ID NO:18, 15, 21, 24, 27, 30, 72, 78, 83 and 86; and
iii. a CDR3 sequence of the amino acid sequence selected from the group consisting of: SEQ ID NO:19, 16, 22, 25, 28, 31, 73, 74, 75, 76, 79, 81, 84, 87, 166 and 167.

6. The antibody for the use according to any one of claims 1-5 wherein said antibody comprises:
- as VHCDR1 SEQ ID NO: 8, as VHCDR2 SEQ ID NO: 5, as VHCDR3 SEQ ID NO: 6, as VLCDR1 SEQ ID NO: 17, as VLCDR2 SEQ ID NO: 18 and as VLCDR3 SEQ ID NO: 19 or Kabat, IMGT, Chothia, AbM, or Contact CDRs of TC01 or of TC05 or
- as VHCDR1 SEQ ID NO: 8, as VHCDR2 SEQ ID NO: 5, as VHCDR3 SEQ ID NO: 6, as VLCDR1 SEQ ID NO: 17, as VLCDR2 SEQ ID NO: 18 and as VLCDR3 SEQ ID NO: 166 or Kabat, IMGT, Chothia, AbM, or Contact CDRs of TC03 or
- as VHCDR1 SEQ ID NO: 8, as VHCDR2 SEQ ID NO: 5, as VHCDR3 SEQ ID NO: 6, as VLCDR1 SEQ ID NO: 17, as VLCDR2 SEQ ID NO: 18 and as VLCDR3 SEQ ID NO: 167 or Kabat, IMGT, Chothia, AbM, or Contact CDRs of TC04 or
- as VHCDR1 SEQ ID NO: 68, as VHCDR2 SEQ ID NO: 69, as VHCDR3 SEQ ID NO: 70, as VLCDR1 SEQ ID NO: 85, as VLCDR2 SEQ ID NO: 86 and as VLCDR3 SEQ ID NO: 87, or Kabat, IMGT, Chothia, AbM, or Contact CDRs of TM1.

7. The antibody for the use according to any one of claims 1-6 wherein said antibody comprises:
a. a heavy chain variable domain sequence of the amino acid sequence selected from the group consisting of: SEQ ID NO:32 to SEQ ID NO:37 or of SEQ ID NO:88 to SEQ ID NO:95, or of SEQ ID NO:168, SEQ ID NO:169 and SEQ ID NO:170; or
b. a light chain variable domain sequence of the amino acid sequence selected from the group consisting of: SEQ ID NO: 38 to SEQ ID NO:43; or of SEQ ID NO: 96 to SEQ ID NO: 103 or of SEQ ID NO: 171, SEQ ID NO: 172 or SEQ ID NO: 173; or
c. the heavy chain variable domain of (a) and the light chain variable domain of (b).

8. The antibody for the use according to any one of claims 1-7 wherein said antibody is selected from the group consisting of TC01, TC03, TC04, TC05, TA02, TC02, TD01, TE01, TG02, TM1, TE02.1, TE02.2, TE02.3, TE03, TE04, TE07, and TE10 or an antigen-binding fragment thereof, as described in Tables 1-8 or encoded by the nucleotide sequences of Tables 9-10.

9. The antibody for the use according to any one of claims 1-8 wherein said antibody is TC01 comprising SEQ ID NO:33 and SEQ ID NO:39, TC03 comprising SEQ ID NO:168 and SEQ ID NO:171, TC04 comprising SEQ ID NO:169 and SEQ ID NO:172, TC05 comprising SEQ ID NO:170 and SEQ ID NO:173, TA02 comprising SEQ ID NO:32 and SEQ ID NO:38, TC02 comprising SEQ ID NO:34 and SEQ ID NO:40, TD01 comprising SEQ ID NO:35 and SEQ ID NO:41, TE01 comprising SEQ ID NO:36 and SEQ ID NO:42, TG02 comprising SEQ ID NO:37 and SEQ ID NO:43, TE02.1 comprising SEQ ID NO:88 and SEQ ID NO:96, TE02.2 comprising SEQ ID NO:89 and SEQ ID NO:97, TE02.3 comprising SEQ ID NO:90 and SEQ ID NO:98, TE03 comprising SEQ ID NO:91 and SEQ ID NO:99, TE04 comprising SEQ ID NO:92 and SEQ ID NO:100, TE07 comprising SEQ ID NO:93 and SEQ ID NO:101, TE10 comprising SEQ ID NO:94 and SEQ ID NO:102, or TM1 comprising SEQ ID NO:95 and SEQ ID NO:103.

10. The antibody for the use according to any one of claims 1-9 wherein said antibody is a human or a humanized antibody and/or is an IgG2 or IgG4 antibody, preferably an IgG2 kappa antibody, an IgG2 lambda antibody, an IgG4 kappa antibody or an IgG4 lambda antibody.

11. The antibody for the use according to any one of claims 1-10, wherein said antibody comprises a light chain comprising a light variable region comprising or consisting of a sequence of SEQ ID NO.39 and a light constant region comprising or consisting of a sequence of SEQ ID NO.123 and/or a heavy chain comprising a heavy variable region comprising or consisting of a sequence of SEQ ID NO.33 and a heavy constant region comprising or consisting of a sequence of SEQ ID NO.120 or of SEQ ID NO. 180, preferably wherein said antibody comprises a light chain comprising or consisting of a sequence of SEQ ID NO. 4 and/or an heavy chain comprising or consisting of a sequence of SEQ ID NO. 125 or of SEQ ID NO. 126.

12. An isolated polynucleotide comprising at least one sequence that encodes the antibody or antigen binding fragment thereof as disclosed in any one of claims 1-11, preferably said polynucleotide being a cDNA, or a vector comprising said polynucleotide, preferably said vector being selected from the group consisting of a plasmid, a viral vector, a non-episomal mammalian vector, an expression vector and a recombinant expression vector, or an isolated cell comprising said polynucleotide or said vector, preferably said isolated cell being a hybridoma or a Chinese Hamster Ovary (CHO) cell or a Human Embryonic Kidney cell (HEK293), for use in the prevention and/or treatment of colorectal cancer.

13. A pharmaceutical composition comprising an antibody that binds to TMEM219 receptor or an antigen-binding fragment thereof and at least one pharmaceutically acceptable carrier for use in the prevention and/or treatment of colorectal cancer, preferably wherein said antibody that binds to TMEM219 receptor is as defined in any one of claims 5-11, preferably said pharmaceutical composition which further comprises a second therapeutic agent.

14. The antibody for use according to any one of claims 1-11 wherein said antibody is used in combination with at least one further therapeutic agent.

15. The antibody for use according to claim 14 or the pharmaceutical composition for use according to claim 13 wherein said therapeutic agent is an active ingredient which is known to be effective in the prevention and/or treatment of cancer, preferably of colorectal cancer, such as a chemotherapeutic agent or a radiotherapy agent, preferably said therapeutic agent is selected from fluoropyrimidine, oxaliplatin, irinotecan, leucovorin, capecitabine, anti-VEGF monoclonal antibodies or anti-VEGF small molecules, such as bevacizumab or aflibercept, cetuximab or panitumumab, ramucirumab, BRAF-inhibitors, such as encorafenib, 5-aminosalicylic acid, immunomodulators, such as thiopurines, and immune checkpoint inhibitors, such as pembrolizumab.
